(19) Europäisches Patentamt
European Patent Office
Office européen des brevets

(11) EP 4 176 863 B1

(12) EUROPÄISCHE PATENTSCHRIFT

(45) Veröffentlichungstag und Bekanntmachung des Hinweises auf die Patenterteilung:
**17.12.2025 Patentblatt 2025/51**

(21) Anmeldenummer: **22199764.6**

(22) Anmeldetag: **05.10.2022**

(51) Internationale Patentklassifikation (IPC):
***A61K 6/40*** *(2020.01)*

(52) Gemeinsame Patentklassifikation (CPC):
**A61K 6/40**

(54) **HOCHWIRKSAMES, KIESELSÄUREFREIES, LAGERSTABILES DENTALES ÄTZGEL**

HIGH EFFICIENCY, NON-SILICA, SHELF STABLE DENTAL ETCHING GEL

GEL DE GRAVURE DENTAIRE À HAUTE EFFICACITÉ, SANS SILICE, STABLE AU STOCKAGE

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**

(30) Priorität: **04.11.2021 DE 102021128685**

(43) Veröffentlichungstag der Anmeldung:
**10.05.2023 Patentblatt 2023/19**

(73) Patentinhaber: **VOCO GmbH**
**27472 Cuxhaven (DE)**

(72) Erfinder:
- **Kelch, Hauke**
  **27472 Cuxhaven (DE)**
- **Krumme, Wigand**
  **27476 Cuxhaven (DE)**
- **Plaumann, Manfred Thomas**
  **27472 Cuxhaven (DE)**

(56) Entgegenhaltungen:
**WO-A1-2017/017036**
**US-A1- 2012 161 067**
**US-A1- 2013 273 745**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Processed by Luminess, 75001 PARIS (FR)

## Beschreibung

Die vorliegende Erfindung betrifft eine dentale Ätzzusammensetzung, die Verwendung dieser dentalen Ätzzusammensetzung zum Ätzen der Zahnhartsubstanz, eine dentale Ätzzusammensetzung zur Anwendung in einem therapeutischen Verfahren zum Ätzen der Zahnhartsubstanz im Rahmen der Füllungstherapie sowie ein Kit enthaltend eine dentale Ätzzusammensetzung. Soweit für einen erfindungsgemäßen Aspekt (Zusammensetzung; Verwendung; Anwendung in einem therapeutischen Verfahren oder Kit) bestimmte Ausgestaltungen als bevorzugt bezeichnet werden, gelten die entsprechenden Ausführungen jeweils auch für die anderen Aspekte der vorliegenden Erfindung, *mutatis mutandis.* Bevorzugte individuelle Merkmale erfindungsgemäßer Aspekte (wie in den Ansprüchen definiert und/oder in der Beschreibung offenbart) sind miteinander kombinierbar und werden vorzugsweise miteinander kombiniert, sofern sich im Einzelfall für den Fachmann aus dem vorliegenden Text nichts anderes ergibt; der Gegenstand der Erfindung wird durch die Ansprüche definiert.

Dentin und Schmelz als Zahnhartsubstanz

Die Zahnhartsubstanz besteht aus Schmelz und Dentin. Während der Schmelz zu 95 Gew.-% aus anorganischer Substanz, zu 4 Gew.-% aus Wasser und zu 1 Gew.-% aus organischen Matrixbestandteilen besteht, ist das Dentin, auch Zahnbein genannt, weniger stark mineralisiert. Es bildet die Hauptmasse des Zahns, stellt ein vitales Hartgewebe dar und verleiht dem Zahn seine spezifische Gestalt. Das Dentin umschließt die Pulpa und wird koronal von Schmelz und im Wurzelbereich von Zement überzogen. Das Dentin entsteht aus der Zahnpapille und ist in seiner chemischen Zusammensetzung mit dem Knochen vergleichbar. Es unterscheidet sich grundsätzlich vom Schmelz. Das Dentin besteht zu 70 Gew.-% aus anorganischen Bestandteilen, vor allem aus Hydroxylapatit, zu ca. 20 Gew.-% aus organischen Bestandteilen und zu etwa 10 Gew.-% aus Wasser. Infolge seines hohen Anteils an organischen Substanzen ist Dentin hochelastisch und verformbar.

Der mineralisierte Teil enthält im Wesentlichen Calcium und Phosphor, variable Konzentrationen an Fluorid, geringe Mengen an Carbonaten und Magnesium sowie einige Spurenelemente. Die organische Matrix besteht zu über 90% aus Kollagen vom Typ I. Der Rest an organischen Substanzen setzt sich aus nicht kollagener Grundstruktur zusammen, wie es beispielsweise Proteine, Lipide, Zitrate und Laktate sind.

In seinem morphologischen Aufbau setzt sich Dentin aus den Dentinkanälchen samt periodontoblastischem Raum, den Odontoblasten mit ihren Fortsätzen, dem peritubulären Dentin, dem intertubulären Dentin und dem Manteldentin zusammen. Intertubuläres Dentin ist das aus Typ-I-Kollagen bestehende Netzwerk, in dem die plättchenförmigen Hydroxylapatit-Kristalle und Dentinliquor eingelagert sind. In den Tubuli sind das peritubuläre Dentin, ein Kollagenfaserschlauch, Odontoblastenfortsätze und Dentinliquor enthalten. Das peritubuläre Dentin, das die Kanalwände auskleidet, ist homogen, dicht und am stärksten von allen Dentinstrukturen mineralisiert.

Die Dentinkanälchen nehmen an Zahl und Durchmesser von der Pulpa zur Schmelz-Dentin-Grenze ab. Sind es an der Pulpa-Dentin-Grenze im Durchschnitt 45.000/$mm^2$, so reduziert sich diese Zahl bei 3 mm Abstand von der Pulpa schon auf 20.000/$mm^2$. Der Durchmesser vermindert sich von 2 bis 3 $\mu$m an der Pulpa auf 0,5 bis 0,9 $\mu$m an der Schmelz-Dentingrenze.

Die Odontoblasten, also die Dentin produzierenden Zellen des Zahns, liegen an der inneren Dentinoberfläche. Sie sind nach ihrer Differenzierung nicht mehr teilungsfähig, sind aber zeitlebens in der Lage, Sekundär- und Tertiärdentin zu bilden. Die Odontoblastenfortsätze verlaufen in den Dentinkanälchen. Jeder Fortsatz ist von Gewebeflüssigkeit, dem Dentinliquor, eingehüllt, das den periodontoblastischen Raum ausfüllt. Die Fortsätze durchsetzen das gesamte Dentin und können bis zu 5.000 $\mu$m lang sein. Seitenäste, die ins intertubuläre Dentin hineinreichen, stehen mit den lateralen Ästen der Nachbarfortsätze in Kontakt. Der zwischen den Odontoblasten gelegene periodontoblastische Raum besteht zum größten Teil aus Gewebeflüssigkeit. Das intertubuläre Dentin trennt die einzelnen Dentinkanälchen. Es ist weniger mineralisiert als das peritubuläre Dentin.

Bei der Präparation eines Zahns kommt es zwangsläufig zur Öffnung der Dentinkanälchen. Es entsteht eine offene Dentinwunde, die aufgrund des Pulpeninnendrucks Dentinliquor entlang der Dentinkanälchen nach außen strömen lässt. Dieses Phänomen wird auch als instrinsische Feuchtigkeit bezeichnet. Aus diesem Grund lässt sich Dentin in vivo nicht absolut trocknen.

Wird Dentin präpariert (beispielsweise mit rotierenden Instrumenten), so entsteht eine 1,5 $\mu$m dicke Schmierschicht (smear layer), die aus 0,5 bis 1,5 $\mu$m großen Partikeln aus Zahnhartsubstanz, Kollagen-, Blut- und Speichelbestandteilen, sowie Bakterien und deren Stoffwechselprodukten besteht. Durch diese Schicht kommt es einerseits zu einer Verpfropfung der Dentinkanälchen, andererseits überzieht die Schmierschicht flächig das präparierte Dentin, was die Permeabilität des Dentins herabsetzt. Diese Schmierschicht ist weder abspülbar, noch mechanisch zu entfernen. Sie erschwert somit die Adaptation der Restaurationsmaterialien auf der Zahnoberfläche und beeinträchtigt die Haftung von Kunststoffen. Die Schmierschicht kann jedoch durch chemische Vorbehandlung des Dentins entfernt werden.

Demgegenüber besteht die anorganische Substanz des Schmelzes hauptsächlich aus Calciumphosphat in der

Form von Hydroxylapatit [$Ca_{10}(PO_4)_6(OH)_2$], welches jedoch durch Einschlüsse von Carbonat, Fluorid, Natrium, Magnesium, Kalium sowie anderer Ionen nicht als stöchiometrisch reines Material angesehen werden kann. Durch interne Substitutionsreaktionen kann Fluorapatit oder fluoridiertes Hydroxylapatit gebildet werden. Die Kristallstrukturen dieser Verbindungen sind säurestabiler als die von reinem Hydroxylapatit.

Der Anteil der anorganischen Verbindungen schwankt je nach Analysenmethode und Entnahmestelle zwischen 93 und 98 Gew.-%. Das Wasser als zweitgrößter Bestandteil variiert zwischen 1,5 und 4 Gew.-%. Aufgrund der unterschiedlichen Konzentrationen der Schmelzzusammensetzung an den verschiedenen Stellen des Zahns nimmt die Menge von Fluorid, Eisen, Zinn, Chlor und Calcium von der Oberfläche in die Tiefe hin ab, wobei an der Schmelz-Dentin Grenze die Fluoridkonzentration wieder zunimmt. Die Konzentrationen von Wasser, Carbonat, Magnesium und Natrium nehmen hingegen von der Schmelzoberfläche zur Schmelz-Dentin-Grenze zu.

Das Wasser liegt sowohl kristallin, gebunden als Hydratationshülle an den Apatitkristallen, als auch lose, fixiert an der organischen Schmelzmatrix, vor. Das lose gebundene Wasser kann unter Erwärmung verdampfen und unter Feuchtigkeitszufuhr wieder aufgenommen werden. Bei diesem Flüssigkeitsstrom wirkt der Schmelz als Molekularsieb, wobei Ionen sowohl aus dem Schmelz heraus, als auch in ihn hineingelangen können.

Die Apatitkristalle weisen einen hexagonalen Querschnitt auf und sind im Durchschnitt 169 nm lang, 40 bis 70 nm breit und 26 nm dick. Obwohl sie chemisch ebenfalls Calciumphosphate des Apatit-Typs darstellen, sind sie sehr viel größer als Kristallite gleichen Typs aber anderer biologischer Herkunft. Etwa 100 Schmelzkritallite liegen im Querschnitt zusammengefügt und bilden die Schmelzprismen oder -stäbe, die sich von der Schmelz-Dentin-Grenze bis zur Oberfläche erstrecken. Die Kristallite im Kern der Prismen sind mit ihrer Längsachse parallel zur Längsachse des jeweiligen Prismas ausgerichtet.

Die Gesamtheit der Kristallite ist in eine gelartige, organische Matrix eingebettet. Die organischen Substanzen des Schmelzes sind vorwiegend Proteine, Lipide sowie Spuren von Kohlenhydraten und organischen Säuren. Alle Kristallite sind zudem von einer Hydratationsschale umgeben.

Die Prismen ihrerseits liegen eingebettet in einer zwischenprismatischen Substanz, die auch aus Schmelzkristalliten gebildet wird. Zwischen Prismen und zwischenprismatischen Zonen gibt es keine Unterschiede bezüglich des anorganischen Gehalts, beide bestehen aus dicht aneinandergelagerten Kristalliten. Die mikroskopisch verifizierbare Strukturierung in prismatische und nichtprismatische Anteile ist lediglich eine Folge der Kristallanordnung. Die Kristallite der zwischenprismatischen Substanz bilden mit der Längsachse der Prismen fast einen rechten Winkel.

Der Schmelz ist für Ionen, Wasser, Farbstoffe und Alkohol nur begrenzt durchgängig. Er besitzt einen hohen Elastizitätsmodul sowie eine geringe Zugfestigkeit.

Sowohl Schmelz als auch Dentin sind somit hochkomplexe Strukturen, die hinsichtlich der dentalen Adhäsivtherapie auch unterschiedliche Betrachtungsweisen verlangen.

Schmelzhaftung

Die Haftung von Kunststoffen am Zahnschmelz beruht vorwiegend auf mikromechanischer Retention und zu einem geringeren Teil auf chemischer Adhäsion. Das Prinzip der überwiegend mechanischen Schmelzhaftung wurde 1955 erstmals beschrieben und ist heute unter der Bezeichnung "Säureätztechnik" oder "Schmelz-Ätz-Technik" ein Standardverfahren bei adhäsiven Restaurationen in der zahnärztlichen Praxis. Aufgrund der unterschiedlichen Löslichkeit der einzelnen Schmelzprismenstrukturen kann mit 30 bis 40%iger Phosphorsäure ein mikroretentives Ätzmuster erzielt werden. In dieses Ätzmuster des Schmelzes kann als Haftvermittler ein niedrigviskoser Kunststoff eindringen und damit den Verbund mit dem Füllungskomposit über eine gute Verzahnung sicherstellen. Im Falle einer Fissurenversiegelung reicht das durch die Schmelzätzung erzeugte Muster aus um auch ohne einen Haftvermittler einen ausreichenden Verbund zwischen Schmelz und dem Versiegelungsmaterial zu erreichen.

Bei kleinen Füllungen im Schmelzbereich ist die mikromechanische Haftung so gut, dass die Polymerisationsschrumpfung komplett abgefangen werden kann. Um den adhäsiven Verbund nicht zu überlasten, wird bei größeren Füllungen durch schichtweise Applikation und separates Aushärten versucht, die Schrumpfung des Materials zu kompensieren. Im Allgemeinen reicht die Haftfestigkeit am Schmelz aus, um das Auftreten von Randspalten durch die Polymerisation zu verhindern.

Während des Einsatzes der Phosphorsäure kommt es zu einer Umwandlung des Schmelzapatits in Brushit und zur Entstehung eines unspezifischen retentiven Ätzmusters, dessen Strukturen als Zotten, Tags, Spalträume, Ausstülpungen oder Mikroporen beschrieben werden. Aus der enormen Oberflächenvergrößerung resultiert eine Erhöhung der Oberflächenenergie und somit eine Steigerung der Benetzbarkeit des geätzten Schmelzes.

Eine optimale Säurewirkung wird nur dann erreicht, wenn der zu ätzende Schmelzbereich restlos von Plaque und Zahnstein befreit ist. Man benutzt in der Regel eine ca. 35%ige Phosphorsäure als Ätzflüssigkeit, die die oberste Schmelzschicht um 5 bis 10 $\mu$m reduziert und die Prismenstruktur bis in eine Tiefe von 30 $\mu$m freilegt. Mikroskopisch werden drei verschiedene Ätzmustertypen beobachtet:

- vorrangige Demineralisation der zentralen Bereiche der Schmelzprismen
- Demineralisation von peripheren Prismenbereichen
- gleichzeitige Demineralisation der Prismenzentren und der Prismenperipherie

Die Phosphorsäure soll in der Regel ca. 30 Sekunden einwirken und wird dann gründlich abgespült. Der Zahnschmelz wird anschließend getrocknet.

Zur Restauration des Zahns mit hochviskosen Kompositmaterialien ist somit ein Haftvermittler notwendig, um die mikromechanische Verankerung zum Schmelz zu gewährleisten. Dieser Haftvermittler wird auch als Sealer, Liner, Primer, Adhäsiv oder bonding agent bezeichnet.

Dentinhaftung

Die adhäsive Verbindung von hydrophoben Kompositmaterialien und Dentin gestaltet sich durch die tubuläre Mikrostruktur, die intrinsische Feuchtigkeit und den höheren Gehalt an organischem Material im Vergleich zum Schmelz erheblich schwieriger und komplexer. Dennoch hat es hier zahllose Weiterentwicklungen gegeben, so dass es heute möglich ist, auch dentinbegrenzte Bereiche mit Komposit zu versorgen.

Der Haftmechanismus der Dentinadhäsive beruht ebenfalls hauptsächlich auf der mikromechanischen Verankerung mit dem Dentin (sogenannte Hybridschicht). Die Verankerung des Haftvermittlers wird durch Verzahnung und Verkettung nach dem Herauslösen organischer und anorganischer Anteile aus dem Dentin mittels saurer ätzender Präparate erreicht.

Es werden verschiedene Möglichkeiten für die mechanisch retentive Verankerung zwischen dem hydrophoben Kunststoff und der feuchten Dentinoberfläche angegeben:

- Zottenbildung durch polymerisiertes Harz in den Tubuli mit bis zu 50 μm Länge
- Verzahnung in Mikroretentionen demineralisierten Dentins
- Verkettung mit freigelegtem Kollagen mit Einschluss nicht gelösten Apatits unter Bildung einer Hybridschicht

Durch die in die Dentintubuli eingedrungenen Monomergemische bilden sich nach der Aushärtung Kunststoffzapfen, die auch als "Tags" bezeichnet werden. Durch den Verbund der "Tags" mit dem demineralisierten peritubulären Dentin kommt es zu einer verbesserten Verbundfestigkeit.

Aus der Durchdringung der konditionierten Dentinoberfläche mit einem Adhäsiv resultiert nach dessen Härtung eine sogenannte Hybridschicht oder "Kunststoff-Dentin-Interdiffusionszone".

Diese mit Kunststoff durchsetzte Dentinschicht soll stärker zur Dentinhaftung beitragen als die Kunststoff-Tags in den Dentintubuli. Aufgrund der Polymerisationsschrumpfung kleiden die Kunststoffzapfen die Kanälchen nicht wandständig aus und es kommt, bedingt durch die Gegenwart des Dentinliquors, zu einer nicht vollständigen Polymerisation der Zapfen. Der Dentinliquor verhindert auch eine tiefe Penetration des Kunststoffs.

Zu einem geringen Teil scheinen auch chemische Anteile am Haftmechanismus eine Rolle zu spielen. Die reaktive Gruppe am Haftvermittler kann mit den anorganischen Bestandteilen des Dentins (insbesondere $Ca^{2+}$) sowie mit den organischen Gruppen des Kollagens (Amino- und Hydroxylgruppen) wechselwirken.

Total Etch

Die Dentinätzung und damit die Schmierschichtentfernung wird als Konditionierung bezeichnet. Als Ätzmittel kommen EDTA-Lösungen, Phosphorsäure (10 bis 40%ig), Maleinsäure (10%ig), Zitronensäure (10%ig) oder Salpetersäure (2,5%ig) zum Einsatz. Das Konditionierungsmittel soll nach einer definierten Einwirkzeit wieder abgespült werden. Je nach der Konzentration der Säure kommt es zu einer partiellen oder totalen Auflösung der Schmierschicht. Die zusätzliche Demineralisation des Dentins führt durch selektive Entfernung von Calciumphosphaten aus dem oberflächlichen Dentin (1 bis 7,5 μm) zur Freilegung von Kollagenfasern. Dieses Netzwerk von Kollagenfasern hat seine mineralisierte Stütze verloren und fällt nach zu starker Trocknung des Dentins wie ein dichtes Bündel auf das darunterliegende Dentin. Dies ist der Grund, warum heutzutage lediglich Wasserüberschüsse von der Dentinoberfläche entfernt werden, das Dentin jedoch nicht komplett trockengeblasen wird. Durch dieses als "Moist-Bonding-Technik" oder "Wet-Bonding-Technik" bezeichnete Verfahren wird durch das zurückbleibende Wasser ein Offenhalten der interfibrillären Hohlräume im kollagenen Fasergeflecht gewährleistet. Es besteht somit für später aufgetragene hydrophile Monomere die Möglichkeit, das demineralisierte Kollagengeflecht zu durchdringen und - durch nachfolgende Polymerisation - eine

mikromechanische Verankerung zu bewerkstelligen. Es findet gleichzeitig auch eine Freilegung des Tubulisystems sowie eine Ätzung des peritubulären Dentins statt.

In der Regel benutzt man heute eine 35 bis 40%ige Phosphorsäurelösung, die nach der "Totalätztechnik" (total etch) angewandt wird. Hierbei wird eine gleichzeitige Schmelz- und Dentinätzung durchgeführt. Die Säure sollte auf dem Dentin nicht länger als 15 bis 20 Sekunden einwirken, da eine Kollagendenaturierung und eine zu starke Dentinpermeabilität zu vermeiden sind. Dies würde zu einer verminderten Haftvermittlung führen und später nachteilig auf die Pulpa wirken.

Man verfährt heute so, dass mit der Applikation des Ätzgels auf dem Schmelz begonnen wird. Nach einer Einwirkzeit von 15 Sekunden wird die Säure dann weiter auf das Dentin aufgetragen, wo sie weitere 15 Sekunden einwirken kann. Die insgesamt 30 Sekunden Einwirkzeit am Schmelz ist notwendig, um ein genügendes Ätzmuster zu erreichen.

Am Beginn einer klinisch erfolgreichen dentalen adhäsiven Therapie steht somit die Konditionierung von Schmelz und Dentin mit einem Ätzmittel.

Die Säure wurde ursprünglich mit Wasser verdünnt, um die gewünschte Konzentration zu erhalten. Jedoch entsteht so eine flüssige Lösung, die man nicht zielgenau auftragen kann. Die wässrigen Säurelösungen haben zudem den Nachteil, dass sie unkontrolliert über die Zahnoberflächen ablaufen und - falls der Zahnarzt keinen Kofferdam zur Trockenlegung verwendet - leicht das Weichgewebe des Mundes angreifen und somit die Patienten schwer verletzen können.

Damit ausschließlich die beabsichtigte Fläche punktgenau geätzt werden kann, sollte das Ätzmittel eine höhere Viskosität und idealerweise einen thixotropen Effekt aufweisen. Um die Viskosität von Ätzmitteln anzuheben, wird häufig Kieselsäure hinzugefügt, so dass ein Gel entsteht. Das Ätzgel kann zielgenau aufgetragen und ein Verlaufen verhindert werden.

Der Begriff "Gel" ist scharf definiert, für ihn gilt

$$\tan \delta < 1,$$

wobei der Verlustfaktor $\tan \delta = G''/G'$ (Verlustmodul/Speichermodul) ist.

In US 4,802,950 von T.P. Croll, betitelt "Enamel bonding etchant and procedure" wird ein pastöses Ätzgel offenbart, umfassend eine wässrige Lösung mit 35% bis 50% Phosphorsäure, pyrogene Kieselsäure und abrasive Siliciumcarbid Partikel. Hier wird die Kieselsäure als Verdickungsmittel eingesetzt, um ein Gel zu erhalten.

Auch in US 6,753,001 B2, bzw. US 6,537,563 B2 von Pentron, betitelt "Dental acid etchant composition and method of use" wird die Kieselsäure zur Herstellung eines Ätzgels verwendet. In diesen Patenten wird eine Zusammensetzung geschützt, die aus einer wässrigen Säurelösung und einem kolloidalen, nanoskaligen Kieselsäuresol in einer Menge von 3 bis 20 Gew.-% bezogen auf die Gesamtzusammensetzung, besteht.

Allerdings weist die Kieselsäure den bedeutenden Nachteil einer geringen Wasserretention auf. Dies führt dazu, dass Wasser verdunsten und sich somit die Säurekonzentration und die Viskosität und/oder die Konsistenz des Ätzmittels im Laufe der Zeit erhöhen kann. Dies führt unweigerlich zu Problemen in der klinischen Praxis. Daher ist es vorteilhaft ein Ätzgel zu verwenden, welches keine Kieselsäure enthält.

Ätzzusammensetzungen ohne Kieselsäure sind bereits aus dem Stand der Technik bekannt.

In US 5,954,996 von Centrix, betitelt "Dental etch and packaging therefore" wird die Zusammensetzung eines Ätzmittels beansprucht, die eine Säure und wasserfreies Glycerin in einer Menge von 10 bis 40 Gew.-% bezogen auf die Zusammensetzung, umfasst. Da bei dieser Zusammensetzung kein überschüssiges Wasser vorhanden ist, verändert sich die Säurekonzentration und die Viskosität mit der Zeit nicht. Allerdings ist dieses Ätzmittel kein Gel, sondern eine Flüssigkeit. Weiterhin stellt sich die Frage, wie diese Zusammensetzung in Abwesenheit eines protischen Lösungsmittels (Wasser) ein Ätzmuster bewirken soll, wenn der Zahnarzt die betreffende Stelle mit Kofferdam trockenlegt.

In US 2012/0161067 A1 von der Far Eastern New Century Corporation, betitelt "Dental etching gel composition and method of use thereof" wird zur Erhöhung der Viskosität Carboxymethylcellulose eingesetzt. Die beanspruchte Zusammensetzung besteht aus einer 37%igen wässrigen Phosphorsäurelösung und Carboxymethylcellulose in Mengen von 0,5 bis 7 Gew.-%, wobei die Viskosität der Carboxymethylcellulose etwa 100 bis etwa 2000 cPs beträgt, wenn diese in einer wässrigen Lösung mit 1 Gew.-% gelöst ist und einen durchschnittlichen Substitutionsgrad von Natriumsalzen in der molekularen Formel von etwa 21% bis 33% aufweist. Man würde erwarten, dass die Säure die Cellulose im Laufe der Zeit angreift und abbaut.

In US 6,312,667 B1 betitelt "Methods of etching hard tissue in the oral environment" wird ein Ätzmittel offenbart, das 17 bis 40 Gew.-% Polyoxyalkylenpolymer enthält. Die Zusammensetzungen zeigen einen Viskositätsanstieg bei erhöhter Temperatur.

In US 6,027,341 von Peridoc AB, betitelt "Dental cavity conditioning" wird eine Zusammensetzung beansprucht, in der das Dentin mit EDTA und der Schmelz mit Phosphor- und/oder Zitronensäure geätzt wird. Bevorzugt wird das

Verfahren mit verdickten Zusammensetzungen durchgeführt. Hierzu werden Cellulose und Derivate von Cellulose, Proteine oder Glykoproteine zur Erhöhung der Viskosität eingesetzt. Wie oben ist zu erwarten, dass die Verdickungsmittel im Laufe der Zeit durch die Säuren angegriffen und abgebaut werden.

In WO 2007/131725 A1 und EP 2 108 356 A1 werden salzsäurehaltige Ätzzusammensetzungen zur Behandlung von Schmelzläsionen offenbart.

In US 5,722,833 wird die Konditionierung von dentalen Keramikoberflächen mit flusssäurehaltigen Ätzzusammensetzungen beschrieben.

In WO 2015/142392 A1 betitelt "Dental etchant compositions comprising one or more dentin collagen crosslinking agents" werden dentale Ätzzusammensetzungen offenbart. Diese können als Säurekomponente Phosphorsäure, Maleinsäure oder Zitronensäure enthalten.

Ziel der vorliegenden Erfindung war es somit, lagerstabile, hochwirksame Ätzgele bereitzustellen, die eine ausreichend hohe Viskosität aufweisen, um eine punktgenaue Applikation zu gewähren. Andererseits sollen die eingesetzten Verdickungsmittel die Nachteile aus dem Stand der Technik überwinden. So sollen sie einerseits säurestabil sein und andererseits ein ausreichend hohes Wasserretentionsvermögen aufweisen.

Erfindungsgemäß wurde überraschenderweise gefunden, dass lagerstabile, kieselsäurefreie, hochwirksame Ätzgele erhalten werden können, wenn diese als Verdickungsmittel bestimmte Typen von Urethan-Harnstoff-Verbindungen aufweisen.

Insbesondere wird die Aufgabe gelöst durch eine dentale Ätzzusammensetzung umfassend

A) Phosphorsäure in einer Menge von 10 bis 45 Gew.-%,
B) Wasser in einer Menge von 30 bis 60 Gew.-%,
C) eine oder mehrere Urethan-Harnstoff-Verbindungen in einer Menge von 5 bis 20 Gew.-%,
D) ein oder mehrere Wasser mischbaren Lösungsmittel in einer Menge von 0 bis 20 Gew.-%, und
E) Farbmittel in einer Menge von 0 bis 5 Gew.-%,

jeweils bezogen auf die Gesamtzusammensetzung,

dadurch gekennzeichnet, dass die Urethan-Harnstoff-Verbindungen (C) der Formel

$$R^1\text{-O-C(=O)-NH-}R^2\text{-NH-C(=O)[-NH-}R^3\text{-NH-C(=O)-NH-}R^2\text{-NH-C(=O)]}_x\text{-O}R^1$$

entsprechen, worin

$R^1$ für einen n-Alkyl-Rest mit 4 bis 22 C-Atomen, einen verzweigten Alkyl-Rest mit 4 bis 22 C-Atomen, einen Alkenylrest mit 3 bis 18 C-Atomen, einen Cycloalkylrest mit 3 bis 20 C-Atomen, einen Arylrest mit 6 bis 12 C-Atomen, einen Arylalkylrest mit 7 bis 12 C-Atomen, einen Rest der Formel $C_mH_{2m+1}(O\text{-}C_nH_{2n})_p\text{-}$, $C_mH_{2m+1}(OOC\text{-}C_vH_{2v})_p\text{-}$ oder $R^5\text{-}C_6H_4(O\text{-}C_nH_{2n})_p\text{-}$ mit m = 1 bis 22, n = 2 bis 4, p = 1 bis 15, v = 4 oder 5 und $R^5$ für einen Alkylrest mit 1 bis 12 C-Atomen steht, wobei unterschiedliche Reste $R^1$ gleich oder verschieden sein können,

$R^2$ für einen verzweigten oder unverzweigten Alkylen-Rest mit 4 bis 22 C-Atomen, Alkenylen-Rest mit 3 bis 18 C-Atomen, Alkinylen-Rest mit 2 bis 20 C-Atomen, Cycloalkylen-Rest mit 3 bis 20 C-Atomen, Cycloalkenylen-Rest mit 3 bis 20 C-Atomen, Arylen-Rest mit 6 bis 12 C-Atomen oder Arylalkylen-Rest mit 7 bis 14 C-Atomen steht, wobei unterschiedliche Reste $R^2$ gleich oder verschieden sein können,

$R^3$ für

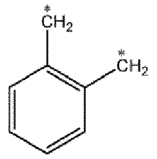 , 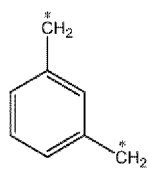 , 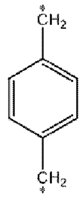 , 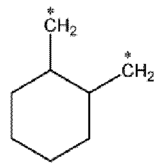 , 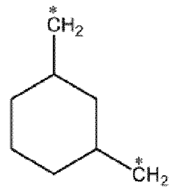 ,

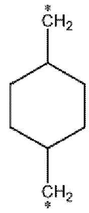

oder

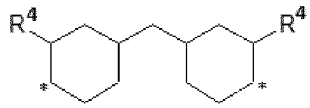

mit $R^4 = CH_3$ oder H, wobei unterschiedliche Reste $R^3$ gleich oder verschieden sein können, und x für eine ganze Zahl von 1 bis 100 steht.

Geeignete Urethan-Harnstoff-Verbindungen (C) sowie deren Synthesen sind in den Patentschriften EP 0 006 252 B1, EP 1 048 681 B1, EP 1 188 779 B1, EP 1 396 510 B1, EP 2 370 489 B1, EP 2 475 699 B1 und EP 3 328 909 B1 beschrieben.

In einer besonders bevorzugten Ausführungsform steht

$R^1$ für einen n-Alkyl-Rest mit 4 bis 10 C-Atomen, einen verzweigten Alkyl-Rest mit 4 bis 10 C-Atomen, einen Alkenylrest mit 3 bis 10 C-Atomen, einen Cycloalkylrest mit 3 bis 10 C-Atomen, einen Arylrest mit 6 bis 12 C-Atomen, einen Arylalkylrest mit 7 bis 12 C-Atomen, einen Rest der Formel $C_mH_{2m+1}(O-C_nH_{2n})_p$-, $C_mH_{2m+1}(OOC-C_vH_{2v})_p$- oder $R^5$-$C_6H_4(O-C_nH_{2n})_p$- mit m = 1 bis 10, n = 2 bis 4, p = 1 bis 15, v = 4 oder 5 und $R^5$ für einen Alkylrest mit 1 bis 12 C-Atomen, bevorzugt für einen n-Alkyl-Rest mit 4 bis 10 C-Atomen, einen verzweigten Alkyl-Rest mit 4 bis 10 C-Atomen, einen Rest der Formel $C_mH_{2m+1}(O-C_nH_{2n})_p$- oder $C_mH_{2m+1}(OOC-C_vH_{2v})_p$-mit m = 1 bis 10, n = 2 bis 4, p = 1 bis 15 und v = 4 oder 5, wobei unterschiedliche Reste $R^1$ gleich oder verschieden sein können,

$R^2$ für

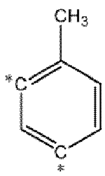

,

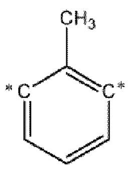

,

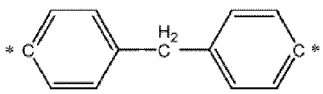

,

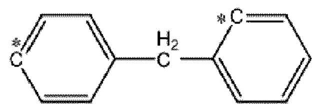

,

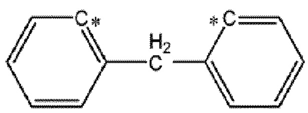

,

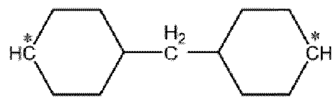

,

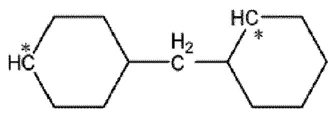

,

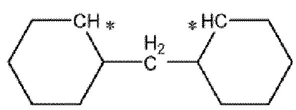

oder $-(CH_2)_w$- mit w = 2 bis 10, bevorzugt für

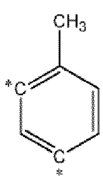

oder

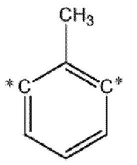

,

wobei unterschiedliche Reste $R^2$ gleich oder verschieden sein können,

$R^3$ für

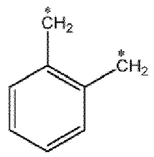

,

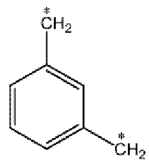

oder

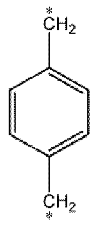

,

wobei unterschiedliche Reste $R^3$ gleich oder verschieden sein können, und

x für eine ganze Zahl von 1 bis 20, bevorzugt 1 bis 10.

Bei der Synthese der Urethan-Harnstoff-Verbindungen (C) wird vorteilhaft zunächst ein Alkohol mit einem Diisocyanat zu einem Monoaddukt umgesetzt.

$$R^1\text{-OH} + \text{OCN-}R^2\text{-NCO} \rightarrow R^1\text{-O-C(=O)-NH-}R^2\text{-NCO}$$

Die Umsetzung erfolgt vorzugsweise in Abwesenheit von Lösungsmitteln. Um möglichst vollständig zum Monoisocyanataddukt zu gelangen, wird vorteilhaft mit einem 1,5- bis 5-fachen Überschuss an Diisocyanat gearbeitet, welches nach erfolgter Umsetzung wieder abdestilliert werden kann.

Das Monoaddukt wird anschließend mit einem Diamin zur Urethan-Harnstoff-Verbindungen (C) umgesetzt.

$$2\ R^1\text{-O-C(=O)-NH-}R^2\text{-NCO} + H_2N\text{-}R^3\text{-}NH_2 \rightarrow R^1\text{-O-C(=O)-NH-}R^2\text{-NH-C(=O)-NH-}R^3\text{-NH-C(=O)-NH-}R^2\text{-NH-C(=O)-O}R^1$$

Ist noch überschüssiges Diisocyanat vorhanden, so gelangt man zu höhermolekularen Urethan-Harnstoff-Verbindungen.

$$2\ R^1\text{-O-C(=O)-NH-}R^2\text{-NCO} + (x\text{-}1)\ \text{OCN-}R^2\text{-NCO} + x\ H_2N\text{-}R^3\text{-}NH_2 \rightarrow R^1\text{-O-C(=O)-NH-}R^2\text{-NH-C(=O)[-NH-}R^3\text{-NH-C(=O)-NH-}R^2\text{-NH-C(=O)]}_x\text{-O}R^1$$

Diese Umsetzung erfolgt vorzugsweise in aprotischen Lösungsmitteln, bevorzugt in DMSO, und kann vorteilhaft in Gegenwart von Lithiumsalzen, bevorzugt Lithiumchlorid, durchgeführt werden. Der Anteil der Urethan-Harnstoff-Verbindungen in der Lösung beträgt vorzugsweise 10 bis 75 Gew.-%, bevorzugt 40 bis 60 Gew.-%.

In einer bevorzugten Ausführungsform umfasst die dentale Ätzzusammensetzung zusätzlich ein oder mehrere mit Wasser mischbare Lösungsmittel (D).

Die mit Wasser mischbaren Lösungsmittel (D) sind vorzugsweise ausgewählt aus der Gruppe bestehend aus Ethanol, Propan-1-ol, Propan-2-ol, Butan-1-ol, Butan-2-ol, 2-Methylpropan-1-ol, 2-Methylpropan-2-ol, Glycerin, Ethylenglycol, Propylenglycol, Butylenglycol, Diethylenglycol, Polyethylenglycol, Polypropylenglycol, 2-Butoxyethan-1-ol, DMSO und Aceton, bevorzugt ausgewählt aus der Gruppe bestehend aus Ethanol, Propan-1-ol, Propan-2-ol, Glycerin, Polyethylenglycol, Polypropylenglycol und DMSO.

In einer bevorzugten Ausführungsform umfasst die dentale Ätzzusammensetzung zusätzlich Farbmittel (E).

Die Farbmittel (E) sind vorzugsweise ausgewählt aus der Gruppe bestehend aus Farbstoffen, organischen Farbpigmenten und anorganischen Farbpigmenten, bevorzugt aus Farbstoffen, besonders bevorzugt aus Phenothiazin-Farbstoffen,
und/oder
bei den Farbmitteln (E) handelt es sich um blaue, grüne oder rote, bevorzugt um blaue, Farbmittel.

In einer bevorzugten Ausführungsform umfasst die dentale Ätzzusammensetzung

A) in einer Menge von 30 bis 42 Gew.-%,
B) in einer Menge von 40 bis 60 Gew.-%,
C) in einer Menge von 5 bis 15 Gew.-%,
D) in einer Menge von 1 bis 15 Gew.-%, und
E) in einer Menge von 0,0001 bis 1 Gew.-%,

jeweils bezogen auf die Gesamtzusammensetzung.
Da die Anwesenheit von anorganischen Feststoffen, insbesondere Kieselsäure wie im Stand der Technik, die oben beschriebenen Nachteile hat, ist in einer besonderen Ausführungsform die dentale Ätzzusammensetzung im Wesentlichen frei von pyrogener Kieselsäure, bevorzugt im Wesentlichen frei von Silicapartikeln, besonders bevorzugt im Wesentlichen frei von anorganischen Feststoffen.
Im Wesentlich frei von bedeutet, dass im Rahmen der technischen Herstellungsmöglichkeiten der Gehalt an pyrogener Kieselsäure, Silicapartikeln bzw. anorganischen Feststoffen so niedrig ist, dass die beschriebenen nachteiligen Effekte nicht auftreten. Vorzugsweise bedeutet im Wesentlichen frei von daher einen Gehalt an pyrogener Kieselsäure, Silicapartikeln bzw. anorganischen Feststoffen von kleiner 1 Gew.-%, bevorzugt kleiner 0.5 Gew.-%, besonders bevorzugt von kleiner 0.1 Gew.-%, jeweils bezogen auf die Gesamtzusammensetzung. Ganz besonders bevorzugt sind Zusammensetzungen, die gar keine pyrogene Kieselsäure, Silicapartikel bzw. anorganischen Feststoffe enthalten. Nicht als anorganische Feststoffe gelten gelöste anorganische Substanzen, insbesondere die in der Synthese der Urethan-Harnstoff-Verbindungen eingesetzten Lithiumsalze.
In einer bevorzugten Ausführungsform enthält die dentale Ätzzusammensetzung außer den Bestandteilen (A), (B), (C), (D) und (E) keine weiteren Bestandteile.
Die erfindungsgemäßen dentalen Ätzzusammensetzungen zeichnen sich durch ihren Gelcharakter und ihre für die Applikation optimale Viskosität aus. Insbesondere weisen die dentalen Ätzzusammensetzungen einen Verlustfaktor tan $\delta$ von kleiner 1 und/oder eine Viskosität im Bereich von 0.1 bis 200 Pa*s, bevorzugt von 0.5 bis 150 Pa*s, besonders bevorzugt von 1 bis 100 Pa*s, ganz besonders bevorzugt von 1 bis 50 Pa*s, auf.
Die Werte für tan $\delta$ und die Viskosität beziehen sich auf die weiter unten in der Beschreibung aufgeführte Messmethode und gelten sowohl für den Auswertepunkt am Ende der Phase I als auch für den Auswertepunkt am Ende der Phase IV.
Die erfindungsgemäßen dentalen Ätzzusammensetzungen zeichnen sich darüber hinaus auch durch ihre gute Lagerstabilität aus. So bleiben der Gelcharakter und die für die Applikation optimale Viskosität auch während der Lagerung weitgehend erhalten. Insbesondere weisen die dentalen Ätzzusammensetzungen vorzugsweise auch nach Lagerung über 6 Monate bei 23 °C einen Verlustfaktor tan $\delta$ von kleiner 1 und/oder eine Viskosität im Bereich 0.1 bis 200 Pa*s, bevorzugt von 0.5 bis 150 Pa*s, besonders bevorzugt von 1 bis 100 Pa*s, ganz besonders bevorzugt von 1 bis 50 Pa*s, auf.
Besonders bevorzugt weisen die dentalen Ätzzusammensetzungen auch nach Lagerung über 12 Monate bei 23 °C, bevorzugt 18 Monate bei 23 °C, besonders bevorzugt 24 Monate bei 23 °C, einen Verlustfaktor tan $\delta$ von kleiner 1 und/oder eine Viskosität im Bereich von 0.1 bis 200 Pa*s, bevorzugt von 0.5 bis 150 Pa*s, besonders bevorzugt von 1 bis 100 Pa*s, ganz besonders bevorzugt von 1 bis 50 Pa*s, auf
und/oder

nach Lagerung über 6 Monate bei 37 °C, bevorzugt 12 Monate bei 37 °C, einen Verlustfaktor tan $\delta$ von kleiner 1 und/oder eine Viskosität im Bereich von 0.1 bis 200 Pa*s, bevorzugt von 0.5 bis 150 Pa*s, besonders bevorzugt von 1 bis 100 Pa*s, ganz besonders bevorzugt von 1 bis 50 Pa*s, auf
und/oder

nach Lagerung über 12 Monate bei 23 °C, bevorzugt 18 Monate bei 23 °C, besonders bevorzugt 24 Monate bei 23 °C, eine Viskosität, die maximal um $\pm$50%, bevorzugt maximal um $\pm$35%, besonders bevorzugt maximal um $\pm$20%, von der Viskosität vor Lagerung abweicht.

Ein weiterer Aspekt der vorliegenden Erfindung ist die Verwendung einer dentalen Ätzzusammensetzung, wie vorstehend beschrieben, zum Ätzen der Zahnhartsubstanz.
In einer bevorzugte Ausführungsform umfasst diese Verwendung die Schritte

i) optional Trockenlegung der zu behandelnden Zähne, vorzugsweise mit Kofferdam,
ii) Auftragen der dentalen Ätzzusammensetzung auf die zu behandelnde Zahnhartsubstanz,
iii) Einwirken lassen der dentalen Ätzzusammensetzung unter Erzielung einer Ätzwirkung an der Zahnhartsubstanz,

iv) Abspülen der dentalen Ätzzusammensetzung,
v) Auftragen einer dentalen Primer- und/oder Adhäsivzusammensetzung auf die geätzte Zahnhartsubstanz,
vi) optional Polymerisation der dentalen Primer- und/oder Adhäsivzusammensetzung,
vii) Auftragen einer dentalen Restaurationszusammensetzung und
viii) Polymerisation der dentalen Restaurationszusammensetzung.

Die vorstehenden Erläuterungen zu den bevorzugten dentalen Ätzzusammensetzungen gelten ebenso für deren Verwendung.
Ein weiterer Aspekt der vorliegenden Erfindung ist eine dentale Ätzzusammensetzung, wie vorstehend beschrieben, zur Anwendung in einem therapeutischen Verfahren zum Ätzen der Zahnhartsubstanz im Rahmen der Füllungstherapie.
In einer bevorzugte Ausführungsform handelt es sich dabei um eine dentale Ätzzusammensetzung, wie vorstehend beschrieben, zur Anwendung in einem therapeutischen Verfahren umfassend die Schritte

i) optional Trockenlegung der zu behandelnden Zähne, vorzugsweise mit Kofferdam,
ii) Auftragen einer dentalen Ätzzusammensetzung nach einem Ansprüche 1 bis 11 auf die zu behandelnde Zahnhartsubstanz,
iii) Einwirken lassen der dentalen Ätzzusammensetzung unter Erzielung einer Ätzwirkung an der Zahnhartsubstanz,
iv) Abspülen der dentalen Ätzzusammensetzung,
v) Auftragen einer dentalen Primer- und/oder Adhäsivzusammensetzung auf die geätzte Zahnhartsubstanz,
vi) optional Polymerisation der dentalen Primer- und/oder Adhäsivzusammensetzung,
vii) Auftragen einer dentalen Restaurationszusammensetzung und
viii) Polymerisation der dentalen Restaurationszusammensetzung.

Die vorstehenden Erläuterungen zu den bevorzugten dentalen Ätzzusammensetzungen gelten ebenso für deren Anwendung in einem therapeutischen Verfahren.
Ein weiterer Aspekt der vorliegenden Erfindung ist ein Kit enthaltend

- eine dentale Ätzzusammensetzung, wie vorstehend beschrieben,
- eine dentale Primer- und/oder Adhäsivzusammensetzung und
- optional eine dentale Restaurationszusammensetzung.

Die vorstehenden Erläuterungen zu den bevorzugten dentalen Ätzzusammensetzungen gelten ebenso für ein Kit, das diese Ätzzusammensetzung enthält.

Beispiele

Beispiel 1A:

Zu 1.5 mol (261.3 g) 2,4-Toluylendiisocyanat werden über 2 Stunden langsam 0.5 mol (37.1 g) 1-Butanol getropft. Die Temperatur wird dabei zwischen 50 und 55 °C gehalten. Nach beendeter Zugabe wird weiter 3 Stunden bei 50 bis 55 °C gerührt, bis der theoretische NCO-Gehalt von 35.2% erreicht ist. Der Überschuss des Diisocyanates wird im Vakuum (0.1 mbar) bei 150 bis 170 °C abdestilliert. Der NCO-Gehalt beträgt 16.9%, der freie TDI-Gehalt <0.5%.

Beispiel 1B:

Zu 1.5 mol (261.3 g) 2,4-Toluylendiisocyanat werden über 2 Stunden langsam 0.5 mol (103.1 g) Triethylenglycolmono-n-butylether getropft. Die Temperatur wird dabei zwischen 50 und 55 °C gehalten. Nach beendeter Zugabe wird weiter 3 Stunden bei 50 bis 55 °C gerührt, bis der theoretische NCO-Gehalt von 28.8% erreicht ist. Der Überschuss des Diisocyanates wird im Vakuum (0.1 mbar) bei 150 bis 170 °C abdestilliert. Der NCO-Gehalt beträgt 11.0%, der freie TDI-Gehalt <0.5%.

Beispiel 1C:

Zu 1.5 mol (261.3 g) 2,4-Toluylendiisocyanat werden über 2 Stunden langsam 0.5 mol (175.0 g) Methoxypolyethylenglykol (MW 350) getropft. Die Temperatur wird dabei zwischen 50 und 55 °C gehalten. Nach beendeter Zugabe wird

weiter 3 Stunden bei 50 bis 55 °C gerührt, bis der theoretische NCO-Gehalt von 24.1% erreicht ist. Der Überschuss des Diisocyanates wird im Vakuum (0.1 mbar) bei 150 bis 170 °C abdestilliert. Der NCO-Gehalt beträgt 8.0%, der freie TDI-Gehalt <0.5%.

Beispiel 1D:

Zu 1.5 mol (261.3 g) 2,4-Toluylendiisocyanat werden über 2 Stunden langsam 0.5 mol (275.0 g) Methoxypolyethylenglykol (MW 550) getropft. Die Temperatur wird dabei zwischen 50 und 55 °C gehalten. Nach beendeter Zugabe wird weiter 3 Stunden bei 50 bis 55 °C gerührt, bis der theoretische NCO-Gehalt von 19.6% erreicht ist. Der Überschuss des Diisocyanates wird im Vakuum (0,1 mbar) bei 150 bis 170 °C abdestilliert. Der NCO-Gehalt beträgt 5.8%, der freie TDI-Gehalt <0.5%.

Beispiel 1E:

Zu 2.0 mol (348.1 g) 2,4-Toluylendiisocyanat werden über 2 Stunden langsam 0.5 mol (175.0 g) Methoxypolyethylenglykol (MW 350) getropft. Die Temperatur wird dabei zwischen 50 und 55 °C gehalten. Nach beendeter Zugabe wird weiter 3 Stunden bei 50 bis 55 °C gerührt, bis der theoretische NCO-Gehalt von 28.1% erreicht ist. Der Überschuss des Diisocyanates wird im Vakuum (0.1 mbar) bei 150 bis 170 °C abdestilliert. Der NCO-Gehalt beträgt 8.0%, der freie TDI-Gehalt <0.5%.

Beispiel 1F:

Zu 1.0 mol (174.2 g) 2,4-Toluylendiisocyanat werden über 2 Stunden langsam 0.5 mol (175.0 g) Methoxypolyethylenglykol (MW 350) getropft. Die Temperatur wird dabei zwischen 50 und 55 °C gehalten. Nach beendeter Zugabe wird weiter 3 Stunden bei 50 bis 55 °C gerührt, bis der theoretische NCO-Gehalt von 18.0% erreicht ist. Der Überschuss des Diisocyanates wird im Vakuum (0.1 mbar) bei 150 bis 170 °C abdestilliert. Der NCO-Gehalt beträgt 8.0%, der freie TDI-Gehalt <0.5%.

Beispiel 1G:

Zu 1.5 mol (375.4 g) Diphenylmethan-4,4'-diisocyanat werden über 2 Stunden langsam 0.5 mol (37.1 g) 1-Butanol getropft. Die Temperatur wird dabei zwischen 50 und 55 °C gehalten. Nach beendeter Zugabe wird weiter 3 Stunden bei 50 bis 55 °C gerührt, bis der theoretische NCO-Gehalt von 25.5% erreicht ist. Der Überschuss des Diisocyanates wird im Vakuum (0.1 mbar) bei 150 bis 170 °C abdestilliert. Der NCO-Gehalt beträgt 13.0%, der freie MDI-Gehalt <0.5%.

Beispiel 1H:

Zu 1.5 mol (252.3 g) 1,6-Hexamethylendiisocyanat werden über 2 Stunden langsam 0.5 mol (37.1 g) 1-Butanol getropft. Die Temperatur wird dabei zwischen 50 und 55 °C gehalten. Nach beendeter Zugabe wird weiter 3 Stunden bei 50 bis 55 °C gerührt, bis der theoretische NCO-Gehalt von 36.3% erreicht ist. Der Überschuss des Diisocyanates wird im Vakuum (0.1 mbar) bei 150 bis 170 °C abdestilliert. Der NCO-Gehalt beträgt 17.3%, der freie HMDI-Gehalt <0.5%.

Tabelle 1: Beispiele 1A bis 1D

| Beispiel | 1A | 1B | 1C | 1D |
|---|---|---|---|---|
| Diisocyanat | 2,4-Toluylendiisocyanat | 2,4-Toluylendiisocyanat | 2,4-Toluylendiisocyanat | 2,4-Toluylendiisocyanat |
| Alkohol | 1-Butanol | Triethylenglykol Monobutylether | Methoxypolyethylenglykol 350 | Methoxypolyethylenglykol 550 |
| Verhältnis Diisocyanat/ Alkohol | 3 : 1 | 3 : 1 | 3 : 1 | 3 : 1 |
| NCO-Gehalt | 16.9% | 11.0% | 8.0% | 5.8% |

Tabelle 1 (Fortsetzung): Beispiele 1E bis 1H

| Beispiel | 1E | 1F | 1G | 1H |
|---|---|---|---|---|
| Diisocyanat | 2,4-Toluylendiisocyanat | 2,4-Toluylendiisocyanat | Diphenylmethan-4,4'-diisocyanat | 1,6-Hexamethylendiisocyanat |
| Alkohol | Methoxypolyethylenglykol 350 | Methoxypolyethylenglykol 350 | 1-Butanol | 1-Butanol |
| Verhältnis Diisocyanat/ Alkohol | 4 : 1 | 2 : 1 | 3 : 1 | 3 : 1 |
| NCO-Gehalt | 8.0% | 8.0% | 13.0% | 17.3% |

Beispiel 2A:

In 175 g DMSO werden bei 80 °C 17.0 g (0.4 mol) Lithiumchlorid und 34.1 g (0.25 mol) 1,3-Xylylendiamin gelöst. Anschließend werden über eine Stunde 124.2 g von Beispiel 1A zugegeben. Nach erfolgter Zugabe wird für weitere 30 Minuten bei 80 °C gerührt und anschließend auf Raumtemperatur abgekühlt. Der Anteil gelöster Feststoffe in der erhaltenen Urethan-Harnstoff-Lösung beträgt 50%.

Beispiel 2B:

In 224 g DMSO werden bei 80 °C 17.0 g (0.4 mol) Lithiumchlorid und 34.1 g (0.25 mol) 1,3-Xylylendiamin gelöst. Anschließend werden über eine Stunde 190.2 g von Beispiel 1B zugegeben. Nach erfolgter Zugabe wird für weitere 30 Minuten bei 80 °C gerührt und anschließend auf Raumtemperatur abgekühlt. Der Anteil gelöster Feststoffe in der erhaltenen Urethan-Harnstoff-Lösung beträgt 50%.

Beispiel 2C:

In 313 g DMSO werden bei 80 °C 17.0 g (0.4 mol) Lithiumchlorid und 34.1 g (0.25 mol) 1,3-Xylylendiamin gelöst. Anschließend werden über eine Stunde 262.1 g von Beispiel 1C zugegeben. Nach erfolgter Zugabe wird für weitere 30 Minuten bei 80 °C gerührt und anschließend auf Raumtemperatur abgekühlt. Der Anteil gelöster Feststoffe in der erhaltenen Urethan-Harnstoff-Lösung beträgt 50%.

Beispiel 2D:

In 413 g DMSO werden bei 80 °C 17.0 g (0.4 mol) Lithiumchlorid und 34.1 g (0.25 mol) 1,3-Xylylendiamin gelöst. Anschließend werden über eine Stunde 362.1 g von Beispiel 1D zugegeben. Nach erfolgter Zugabe wird für weitere 30 Minuten bei 80 °C gerührt und anschließend auf Raumtemperatur abgekühlt. Der Anteil gelöster Feststoffe in der erhaltenen Urethan-Harnstoff-Lösung beträgt 50%.

Beispiel 2E:

In 313 g DMSO werden bei 80 °C 17.0 g (0.4 mol) Lithiumchlorid und 34.1 g (0.25 mol) 1,3-Xylylendiamin gelöst. Anschließend werden über eine Stunde 262.1 g von Beispiel 1E zugegeben. Nach erfolgter Zugabe wird für weitere 30 Minuten bei 80 °C gerührt und anschließend auf Raumtemperatur abgekühlt. Der Anteil gelöster Feststoffe in der erhaltenen Urethan-Harnstoff-Lösung beträgt 50%.

Beispiel 2F:

In 313 g DMSO werden bei 80 °C 17.0 g (0.4 mol) Lithiumchlorid und 34.1 g (0.25 mol) 1,3-Xylylendiamin gelöst. Anschließend werden über eine Stunde 262.1 g von Beispiel 1F zugegeben. Nach erfolgter Zugabe wird für weitere 30 Minuten bei 80 °C gerührt und anschließend auf Raumtemperatur abgekühlt. Der Anteil gelöster Feststoffe in der erhaltenen Urethan-Harnstoff-Lösung beträgt 50%.

Beispiel 2G:

In 213 g DMSO werden bei 80 °C 17.0 g (0.4 mol) Lithiumchlorid und 34.1 g (0.25 mol) 1,3-Xylylendiamin gelöst. Anschließend werden über eine Stunde 162.2 g von Beispiel 1G zugegeben. Nach erfolgter Zugabe wird für weitere 30 Minuten bei 80 °C gerührt und anschließend auf Raumtemperatur abgekühlt. Der Anteil gelöster Feststoffe in der erhaltenen Urethan-Harnstoff-Lösung beträgt 50%.

Beispiel 2H:

In 172 g DMSO werden bei 80 °C 17.0 g (0.4 mol) Lithiumchlorid und 34.1 g (0.25 mol) 1,3-Xylylendiamin gelöst. Anschließend werden über eine Stunde 121.2 g von Beispiel 1H zugegeben. Nach erfolgter Zugabe wird für weitere 30 Minuten bei 80 °C gerührt und anschließend auf Raumtemperatur abgekühlt. Der Anteil gelöster Feststoffe in der erhaltenen Urethan-Harnstoff-Lösung beträgt 50%.

Beispiel 2I:

In 313 g DMSO werden bei 80 °C 17.0 g (0.4 mol) Lithiumchlorid und 34.1 g (0.25 mol) 1,2-Xylylendiamin gelöst. Anschließend werden über eine Stunde 262.1 g von Beispiel 1C zugegeben. Nach erfolgter Zugabe wird für weitere 30 Minuten bei 80 °C gerührt und anschließend auf Raumtemperatur abgekühlt. Der Anteil gelöster Feststoffe in der erhaltenen Urethan-Harnstoff-Lösung beträgt 50%.

Beispiel 2J:

In 313 g DMSO werden bei 80 °C 17.0 g (0.4 mol) Lithiumchlorid und 34.1 g (0.25 mol) 1,4-Xylylendiamin gelöst. Anschließend werden über eine Stunde 262.1 g von Beispiel 1C zugegeben. Nach erfolgter Zugabe wird für weitere 30 Minuten bei 80 °C gerührt und anschließend auf Raumtemperatur abgekühlt. Der Anteil gelöster Feststoffe in der erhaltenen Urethan-Harnstoff-Lösung beträgt 50%.

Beispiel 2K:

In 315 g DMSO werden bei 80 °C 17.0 g (0.4 mol) Lithiumchlorid und 35.6 g (0.25 mol) 1,3-Bis(aminomethyl) cyclohexan gelöst. Anschließend werden über eine Stunde 262.1 g von Beispiel 1C zugegeben. Nach erfolgter Zugabe wird für weitere 30 Minuten bei 80 °C gerührt und anschließend auf Raumtemperatur abgekühlt. Der Anteil gelöster Feststoffe in der erhaltenen Urethan-Harnstoff-Lösung beträgt 50%.

Beispiel 2L:

In 324 g DMSO werden bei 80 °C 27.6 g (0.4 mol) Lithiumnitrat und 34.1 g (0.25 mol) 1,3-Xylylendiamin gelöst. Anschließend werden über eine Stunde 262.1 g von Beispiel 1C zugegeben. Nach erfolgter Zugabe wird für weitere 30 Minuten bei 80 °C gerührt und anschließend auf Raumtemperatur abgekühlt. Der Anteil gelöster Feststoffe in der erhaltenen Urethan-Harnstoff-Lösung beträgt 50%.

Beispiel 2M:

In 209 g DMSO werden bei 80 °C 17.0 g (0.4 mol) Lithiumchlorid und 34.1 g (0.25 mol) 1,3-Xylylendiamin gelöst. Anschließend werden über eine Stunde 262.1 g von Beispiel 1C zugegeben. Nach erfolgter Zugabe wird für weitere 30 Minuten bei 80 °C gerührt und anschließend auf Raumtemperatur abgekühlt. Der Anteil gelöster Feststoffe in der erhaltenen Urethan-Harnstoff-Lösung beträgt 40%.

Beispiel 2N:

In 470 g DMSO werden bei 80 °C 17.0 g (0.4 mol) Lithiumchlorid und 34.1 g (0.25 mol) 1,3-Xylylendiamin gelöst. Anschließend werden über eine Stunde 262.1 g von Beispiel 1C zugegeben. Nach erfolgter Zugabe wird für weitere 30 Minuten bei 80 °C gerührt und anschließend auf Raumtemperatur abgekühlt. Der Anteil gelöster Feststoffe in der erhaltenen Urethan-Harnstoff-Lösung beträgt 60%.

Tabelle 2: Beispiele 2A bis 2D

| Beispiel | 2A | 2B | 2C | 2D |
|---|---|---|---|---|
| Monoaddukt | 1A | 1B | 1C | 1D |
| Diamin | 1,3-Xylylendiamin | 1,3-Xylylendiamin | 1,3-Xylylendiamin | 1,3-Xylylendiamin |
| Li-Salz | LiCl | LiCl | LiCl | LiCl |
| Feststoffgehalt | 50% | 50% | 50% | 50% |

Tabelle 2 (Fortsetzung): Beispiele 2E bis 2H

| Beispiel | 2E | 2F | 2G | 2H |
|---|---|---|---|---|
| Monoaddukt | 1E | 1F | 1G | 1H |
| Diamin | 1,3-Xylylendiamin | 1,3-Xylylendiamin | 1,3-Xylylendiamin | 1,3-Xylylendiamin |

(fortgesetzt)

| Beispiel | 2E | 2F | 2G | 2H |
|---|---|---|---|---|
| Monoaddukt | 1E | 1F | 1G | 1H |
| Li-Salz | LiCl | LiCl | LiCl | LiCl |
| Feststoffgehalt | 50% | 50% | 50% | 50% |

Tabelle 2 (Fortsetzung): Beispiele 2I bis 2L

| Beispiel | 2I | 2J | 2K | 2L |
|---|---|---|---|---|
| Monoaddukt | 1C | 1C | 1C | 1C |
| Diamin | 1,2-Xylylendiamin | 1,4-Xylylendiamin | 1,3-Bis(aminomethyl)cyclohexan | 1,3-Xylylendiamin |
| Li-Salz | LiCl | LiCl | LiCl | $LiNO_3$ |
| Feststoffgehalt | 50% | 50% | 50% | 50% |

Tabelle 2 (Fortsetzung): Beispiele 2M bis 2N

| Beispiel | 2M | 2N |
|---|---|---|
| Monoaddukt | 1C | 1C |
| Diamin | 1,3-Xylylendiamin | 1,3-Xylylendiamin |
| Li-Salz | LiCl | LiCl |
| Feststoffgehalt | 40% | 60% |

Beispiel 3A:

In einem Becherglas wurden unter Rühren 41.2 g 85%ige Phosphorsäure und 1.0 g PEG-400 und 0.01 g Methylenblau in 39.4 g demineralisiertem Wasser gelöst. Anschließend wurden unter Rühren portionsweise 18.4 g der DMSO-Lösung aus Beispiel 2A zugegeben und für weitere 30 Minuten bei Raumtemperatur gerührt.

Beispiele 3B bis 3N:

Analog zu Beispiel 3A wurden die Ätzgele 3B bis 3N hergestellt, wobei statt der DMSO-Lösung aus Beispiel 2A die DMSO-Lösungen aus Beispielen 2B bis 2N eingesetzt wurden.

Beispiel 3O:

In einem Becherglas wurden unter Rühren 41.2 g 85%ige Phosphorsäure und 1.0 g Glycerin und 0.01 g Methylenblau in 39.4 g demineralisiertem Wasser gelöst. Anschließend wurden unter Rühren portionsweise 18.4 g der DMSO-Lösung aus Beispiel 2A zugegeben und für weitere 30 Minuten bei Raumtemperatur gerührt.

Beispiel 3P:

In einem Becherglas wurden unter Rühren 41.2 g 85%ige Phosphorsäure und 2.0 g Ethanol und 0.01 g Methylenblau in 38.4 g demineralisiertem Wasser gelöst. Anschließend wurden unter Rühren portionsweise 18.4 g der DMSO-Lösung aus Beispiel 2A zugegeben und für weitere 30 Minuten bei Raumtemperatur gerührt.

Beispiel 3Q:

In einem Becherglas wurden unter Rühren 41.2 g 85%ige Phosphorsäure und 0.01 g Methylenblau in 40.4 g demineralisiertem Wasser gelöst. Anschließend wurden unter Rühren portionsweise 18.4 g der DMSO-Lösung aus Beispiel 2A zugegeben und für weitere 30 Minuten bei Raumtemperatur gerührt.

Beispiel 3R:

In einem Becherglas wurden unter Rühren 41.2 g 85%ige Phosphorsäure, 2.5 g PEG-400, 4.5 g Glycerin und 0.01 g Methylenblau in 39.4 g demineralisiertem Wasser gelöst. Anschließend wurden unter Rühren portionsweise 12.4 g der DMSO-Lösung aus Beispiel 2A zugegeben und für weitere 30 Minuten bei Raumtemperatur gerührt.

Beispiel 3S:

In einem Becherglas wurden unter Rühren 41.2 g 85%ige Phosphorsäure, 1.0 g PEG-400 und 1.5 g Glycerin und 0.01 g Methylenblau in 41.3 g demineralisiertem Wasser gelöst. Anschließend wurden unter Rühren portionsweise 15.0 g der DMSO-Lösung aus Beispiel 2A zugegeben und für weitere 30 Minuten bei Raumtemperatur gerührt.

Beispiel 3T:

In einem Becherglas wurden unter Rühren 41.2 g 85%ige Phosphorsäure und 1.0 g Glycerin und 0.01 g Methylenblau in 36.8 g demineralisiertem Wasser gelöst. Anschließend wurden unter Rühren portionsweise 22.0 g der DMSO-Lösung aus Beispiel 2A zugegeben und für weitere 30 Minuten bei Raumtemperatur gerührt.

Beispiel 3U:

In einem Becherglas wurden unter Rühren 41.2 g 85%ige Phosphorsäure und 0.5 g Glycerin und 0.01 g Methylenblau in 33.9 g demineralisiertem Wasser gelöst. Anschließend wurden unter Rühren portionsweise 24.4 g der DMSO-Lösung aus Beispiel 2A zugegeben und für weitere 30 Minuten bei Raumtemperatur gerührt.

Tabelle 3: Beispiele 3A bis 3Q

| | | 3A bis 3N | 3O | 3P | 3Q |
|---|---|---|---|---|---|
| (A)*1 | $H_3PO_4$*1 | 35.02 | 35.02 | 35.02 | 35.02 |
| (B)*2 | Wasser*2 | 45.57 | 45.57 | 44.57 | 46.57 |
| (C)*3 | Urethan-Harnstoff-Verbindung*3 | 9.20 | 9.20 | 9.20 | 9.20 |
| (D)*4 | PEG-400 | 1.00 | | | |
| | Glycerin | | 1.00 | | |
| | Ethanol | | | 2.00 | |
| | DMSO*4 | 9.20 | 9.20 | 9.20 | 9.20 |
| (E) | Methylenblau | 0.01 | 0.01 | 0.01 | 0.01 |
| Gesamt | | 100.00 | 100.00 | 100.00 | 100.00 |

*1 Da in den Beispielen eine 85%ige Phosphorsäure verwendet wurde, wird hier unter (A) nur der tatsächliche Anteil Phosphorsäure angegeben.
*2 Neben dem eingesetzten Wasser wird unter (B) auch der Wasseranteil aus der Phosphorsäure angegeben.
*3 Da es sich bei den Beispielen 2 um eine Lösung der Urethan-Harnstoff-Verbindung handelt, wird unter (C) nur der tatsächliche Anteil der Urethan-Harnstoff-Verbindung angegeben.
*4 Neben etwaigen weiteren mit Wasser mischbaren Lösungsmitteln wird unter (D) auch der Anteil des Lösungsmittels der Lösung der Urethan-Harnstoff-Verbindung angegeben.

Tabelle 3 (Fortsetzung): Beispiele 3R bis 3U

| | | 3R | 3S | 3T | 3U |
|---|---|---|---|---|---|
| (A)*1 | $H_3PO_4$*1 | 35.02 | 35.02 | 35.02 | 35.02 |
| (B)*2 | Wasser*2 | 45.57 | 47.47 | 41.97 | 40.07 |
| (C)*3 | Urethan-Harnstoff-Verbindung*3 | 6.20 | 7.50 | 11.00 | 12.20 |

(fortgesetzt)

| | | 3R | 3S | 3T | 3U |
|---|---|---|---|---|---|
| (D)*4 | PEG-400 | 2.50 | 1.00 | | |
| | Glycerin | 4.50 | 1.50 | 1.00 | 0.50 |
| | DMSO*4 | 6.20 | 7.50 | 11.00 | 12.20 |
| (E) | Methylenblau | 0.01 | 0.01 | 0.01 | 0.01 |
| Gesamt | | 100.00 | 100.00 | 100.00 | 100.00 |

Vergleichsbeispiel 4A:

In einem Becherglas wurden unter Rühren 10.0 g Gummi arabicum und 0.01 g Methylenblau in 48.8 g demineralisiertem Wasser gelöst. Anschließend wurden unter Rühren 41.2 g 85%ige Phosphorsäure zugegeben und für weitere 30 Minuten bei Raumtemperatur gerührt. Es tritt kein verdickender Effekt ein. Die Lösung ist dünnflüssig.

Vergleichsbeispiel 4B:

In einem Becherglas wurden unter Rühren 20.0 g Gummi arabicum und 0.01 g Methylenblau in 38.8 g demineralisiertem Wasser gelöst. Anschließend wurden unter Rühren 41.2 g 85%ige Phosphorsäure zugegeben und für weitere 30 Minuten bei Raumtemperatur gerührt. Es tritt kein verdickender Effekt ein. Die Lösung ist dünnflüssig.

Vergleichsbeispiel 4C:

In einem Becherglas wurden unter Rühren 2.0 g Xanthan Gum und 0.01 g Methylenblau in 56.8 g demineralisiertem Wasser gelöst. Anschließend wurden unter Rühren 41.2 g 85%ige Phosphorsäure zugegeben und für weitere 30 Minuten bei Raumtemperatur gerührt. Die Viskosität beträgt 3.0 Pa*s. Während der Lagerung bei 23 °C steigt die Viskosität innerhalb von 6 Monaten langsam auf 4.2 Pa*s an. Bereits nach einer Lagerdauer von einem Monat bei 23 °C tritt eine deutliche Gasbildung auf.

Vergleichsbeispiel 4D:

In einem Becherglas wurden unter Rühren 5.0 g Xanthan Gum und 0.01 g Methylenblau in 53.8 g demineralisiertem Wasser gelöst. Anschließend wurden unter Rühren 41.2 g 85%ige Phosphorsäure zugegeben und für weitere 30 Minuten bei Raumtemperatur gerührt. Die Viskosität beträgt 6.0 Pa*s. Während der Lagerung bei 23 °C steigt die Viskosität innerhalb von 6 Monaten langsam auf 8.3 Pa*s an. Bereits nach einer Lagerdauer von einem Monat bei 23 °C tritt eine deutliche Gasbildung auf.

Vergleichsbeispiel 4E:

In einem Becherglas wurden unter Rühren 10.0 g Polyvinylalkohol und 0.01 g Methylenblau in 48.8 g demineralisiertem Wasser gelöst. Anschließend wurden unter Rühren 41.2 g 85%ige Phosphorsäure zugegeben und für weitere 30 Minuten bei Raumtemperatur gerührt. Es tritt kaum ein verdickender Effekt ein. Die Lösung ist dünnflüssig.

Vergleichsbeispiel 4F:

In einem Becherglas wurden unter Rühren 20.0 g Polyvinylalkohol und 0.01 g Methylenblau in 38.8 g demineralisiertem Wasser gelöst. Anschließend wurden unter Rühren 41.2 g 85%ige Phosphorsäure zugegeben und für weitere 30 Minuten bei Raumtemperatur gerührt. Es tritt kaum ein verdickender Effekt ein. Die Lösung ist dünnflüssig.

Vergleichsbeispiel 4G:

In einem Becherglas wurden unter Rühren 30.0 g Polyvinylalkohol und 0.01 g Methylenblau in 28.8 g demineralisiertem Wasser gelöst. Anschließend wurden unter Rühren 41.2 g 85%ige Phosphorsäure zugegeben und für weitere 30 Minuten bei Raumtemperatur gerührt. Die Viskosität beträgt 10.0 Pa*s. Das Material lässt sich nicht gut auf die präparierte Zahnoberfläche applizieren. Es wird durch die Bewegung bei der Applikation flüssig und "fließt" vom Zahn.

Vergleichsbeispiel 4H:

In einem Becherglas wurden unter Rühren 2.5 g Hydroxyethylcellulose und 0.01 g Methylenblau in 56.3 g demineralisiertem Wasser gelöst. Anschließend wurden unter Rühren 41.2 g 85%ige Phosphorsäure zugegeben und für weitere 30 Minuten bei Raumtemperatur gerührt. Es tritt nur ein geringer verdickender Effekt ein. Die Lösung ist relativ dünnflüssig.

Vergleichsbeispiel 4I:

In einem Becherglas wurden unter Rühren 5.0 g Hydroxyethylcellulose und 0.01 g Methylenblau in 53.8 g demineralisiertem Wasser gelöst. Anschließend wurden unter Rühren 41.2 g 85%ige Phosphorsäure zugegeben und für weitere 30 Minuten bei Raumtemperatur gerührt. Die Viskosität beträgt 6.5 Pa*s. Während der Lagerung bei 23 °C nimmt die Viskosität innerhalb von einem Monat bereits auf 2.5 Pa*s ab.

Vergleichsbeispiel 4J:

In einem Becherglas wurden unter Rühren 20.0 g Glycerin und 0.01 g Methylenblau in 38.8 g demineralisiertem Wasser gelöst. Anschließend wurden unter Rühren 41.2 g 85%ige Phosphorsäure zugegeben und für weitere 30 Minuten bei Raumtemperatur gerührt. Es tritt kein verdickender Effekt ein. Die Lösung ist dünnflüssig.

Vergleichsbeispiel 4K:

In einem Becherglas wurden unter Rühren 40.0 g Glycerin und 0.01 g Methylenblau in 18.8 g demineralisiertem Wasser gelöst. Anschließend wurden unter Rühren 41.2 g 85%ige Phosphorsäure zugegeben und für weitere 30 Minuten bei Raumtemperatur gerührt. Es tritt kein verdickender Effekt ein. Die Lösung ist dünnflüssig.

Vergleichsbeispiel 4L:

In einem Becherglas wurden unter Rühren 0.01 g Methylenblau in 58.8 g Glycerin gelöst. Anschließend wurden unter Rühren 41.2 g 85%ige Phosphorsäure zugegeben und für weitere 30 Minuten bei Raumtemperatur gerührt. Es tritt kein verdickender Effekt ein. Die Lösung ist dünnflüssig.

Vergleichsbeispiel 4M:

In einem Becherglas wurden unter Rühren 2.5 g Carboxymethylcellulose und 0.01 g Methylenblau in 56.3 g demineralisiertem Wasser gelöst. Anschließend wurden unter Rühren 41.2 g 85%ige Phosphorsäure zugegeben und für weitere 30 Minuten bei Raumtemperatur gerührt. Die Viskosität beträgt 2.5 Pa*s, nimmt aber während der Lagerung merklich ab und das Gel wird flüssiger.

Vergleichsbeispiel 4N:

In einem Becherglas wurden unter Rühren 5.0 g Carboxymethylcellulose und 0.01 g Methylenblau in 53.8 g demineralisiertem Wasser gelöst. Anschließend wurden unter Rühren 41.2 g 85%ige Phosphorsäure zugegeben und für weitere 30 Minuten bei Raumtemperatur gerührt. Die Viskosität beträgt 8.2 Pa*s, nimmt aber während der Lagerung merklich ab und das Gel wird flüssiger.

Vergleichsbeispiel 4O:

In einem Becherglas wurden unter Rühren 0.01 g Methylenblau in 58.8 g demineralisiertem Wasser gelöst. Anschließend wurden unter Rühren 41.2 g 85%ige Phosphorsäure zugegeben. Anschließend wurden 5.0 g Aerosil A200 zugegeben und mit einem Ultra Turrax für 5 Minuten dispergiert. Die Viskosität beträgt 10.4 Pa*s, nimmt aber während der Lagerung merklich zu und das Gel dickt ein.

Vergleichsbeispiel 4P:

In einem Becherglas wurden unter Rühren 0.01 g Methylenblau in 8.8 g demineralisiertem Wasser gelöst. Anschließend wurden unter Rühren zunächst 50.0 g Snowtex ST-O (20% kolloidale Kieselsäure in Wasser; Partikelgröße 10 - 20 nm) und dann 41.2 g 85%ige Phosphorsäure zugegeben und für weitere 30 Minuten bei Raumtemperatur gerührt.

Die Viskosität beträgt 8.8 Pa*s, nimmt aber während der Lagerung merklich zu und das Gel dickt ein.

Tabelle 4: Vergleichsbeispiele 4A bis 4D

| | | 4A | 4B | 4C | 4D |
|---|---|---|---|---|---|
| (A)*1 | $H_3PO_4$*1 | 35.02 | 35.02 | 35.02 | 35.02 |
| (B)*2 | Wasser*2 | 54.97 | 44.97 | 62.97 | 59.97 |
| (X) | Gummi arabicum | 10.00 | 20.00 | | |
| | Xanthan Gum | | | 2.00 | 5.00 |
| (E) | Methylenblau | 0.01 | 0.01 | 0.01 | 0.01 |
| Gesamt | | 100.00 | 100.00 | 100.00 | 100.00 |

Tabelle 4 (Fortsetzung): Vergleichsbeispiele 4E bis 4H

| | | 4E | 4F | 4G | 4H |
|---|---|---|---|---|---|
| (A)*1 | $H_3PO_4$*1 | 35.02 | 35.02 | 35.02 | 35.02 |
| (B)*2 | Wasser*2 | 54.97 | 44.97 | 34.97 | 62.47 |
| (X) | Polyvinylalkohol | 10.00 | 20.00 | 30.00 | |
| | Hydroxyethylcellulose | | | | 2.50 |
| (E) | Methylenblau | 0.01 | 0.01 | 0.01 | 0.01 |
| Gesamt | | 100.00 | 100.00 | 100.00 | 100.00 |

Tabelle 4 (Fortsetzung): Vergleichsbeispiele 4I bis 4L

| | | 4I | 4J | 4K | 4L |
|---|---|---|---|---|---|
| (A)*1 | $H_3PO_4$*1 | 35.02 | 35.02 | 35.02 | 35.02 |
| (B)*2 | Wasser*2 | 59.97 | 44.97 | 24.97 | 6.18 |
| (X) | Hydroxyethylcellulose | 5.00 | | | |
| (D) | Glycerin | | 20.00 | 40.00 | 58.79 |
| (E) | Methylenblau | 0.01 | 0.01 | 0.01 | 0.01 |
| Gesamt | | 100.00 | 100.00 | 100.00 | 100.00 |

Tabelle 4 (Fortsetzung): Vergleichsbeispiele 4M bis 4P

| | | 4M | 4N | 4O | 4P |
|---|---|---|---|---|---|
| (A)*1 | $H_3PO_4$*1 | 35.02 | 35.02 | 35.02 | 35.02 |
| (B)*2,5 | Wasser*2,5 | 62.47 | 59.97 | 59.97 | 54.97 |
| (X) | Carboxymethylcellulose | 2.50 | 5.00 | | |
| | pyrogene Kieselsäure | | | 5.00 | |
| | kolloidale Kieselsäure*6 | | | | 10.00 |
| (E) | Methylenblau | 0.01 | 0.01 | 0.01 | 0.01 |

(fortgesetzt)

| | | 4M | 4N | 4O | 4P |
|---|---|---|---|---|---|
| Gesamt | | 100.00 | 100.00 | 100.00 | 100.00 |

*1 Da in den Beispielen eine 85%ige Phosphorsäure verwendet wurde, wird hier unter (A) nur der tatsächliche Anteil Phosphorsäure angegeben.
*2 Neben dem eingesetzten Wasser wird unter (B) auch der Wasseranteil aus der Phosphorsäure angegeben.
*5 Bei der Verwendung der wässrigen Dispersion kolloidaler Kieselsäure wird unter (B) auch der Wasseranteil aus der Dispersion angegeben.
*6 Unter (X) ist für die kolloidale Kieselsäure nur der $SiO_2$-Anteil angegeben.

Scherverbundfestigkeit:

Die Prüfungen zur Scherverbundfestigkeit wurden entsprechende ISO 29022:2013 durchgeführt. Rinderfrontzähne wurden in einer Epoxid-Matrix in Form eines Zylinders mit Durchmesser d = 2.5 cm eingebettet anschließend wurde die Schmelz- bzw. Dentinfläche exponiert. Die Oberfläche der Zähne wurde standardisiert durch grobes Beschleifen mit Schleifpapier der Körnung P120 (125 ± 1μm) und anschließend feines Beschleifen mit Schleifpapier der Körnung P400 (35 ± 1 μm). Die so präparierte Zahnoberfläche wurde von Verunreinigungen unter fließendem deionisiertem Wasser befreit und anschließend durch einen leichten/kurzzeitig aufgebrachten Strahl aus öl- und wasserfreier Druckluft unmittelbar vor Aufbringung des Ätzmittels von überschüssigem Wasser befreit. Die Zähne dürfen nicht übertrocknet werden, um morphologische Veränderungen der Zahnhartsubstanz zu unterbinden. Das Ätzmittel wird direkt aus der Spritze flächig auf die Zahnhartsubstanz aufgetragen und 30 s (Schmelz) bzw. 15 s (Dentin) unbewegt belassen. Anschließend wird unter fließendem Wasser für mehrere Sekunden abgespült. Der erhaltene geätzte Zahn wird durch einen leichten/kurzzeitig aufgebrachten Strahl aus öl- und wasserfreier Druckluft von überschüssigem Wasser befreit und feucht weiterverwendet. Das Adhäsiv (Futurabond U, VOCO GmbH) wird auf die präparierte Zahnfläche aufgebracht und für 20 s in die Oberfläche einmassiert. Im Adhäsiv enthaltene Lösungsmittel werden durch 5-sekündiges Verblasen mit einem Strahl aus öl- und wasserfreier Druckluft entfernt. Anschließend wird eine Lichthärtung für 10 s durchgeführt (Celalux 2, VOCO GmbH, 420 - 490 nm, 1000 W/$cm^2$). Nach der Aushärtung wird der eingebettete Zahnprobekörper in eine Verbundschraubzwinge inklusive Einsteckform gebracht (entsprechend ISO 29022:2013 - FA. Ultradent Products, South Jordan). Die Einsteckform wird auf die Oberfläche des Zahnes gebracht, auf hinreichende Passung geprüft und mit den Schrauben der Apparatur fixiert. Das Komposit (GrandioSO A1, VOCO GmbH) wird in die Mulde der Einsteckform mit einem Stopfinstrument auf die Verbundfläche aufgebracht und anschließend für 10 Sekunden lichtgehärtet (Celalux 2, VOCO GmbH, 420 - 490 nm, 1000 W/$cm^2$).

Der Verbundprobekörper wird aus der Verbundschraubzwinge entnommen und für (24 ± 2) h in Wasser bei (37 ± 2) °C aufbewahrt. Unmittelbar nach ihrer Entnahme aus dem Wasser erfolgt die Bestimmung der Scherverbundfestigkeit. Dazu werden die Verbundprobekörper in einem Scherversuch an einer Universalprüfmaschine (ZwickRoell GmbH & Co. KG, Ulm) bei einer Querhauptgeschwindigkeit von (1.0 ± 0,1) mm/min und einer Vorkraft von 1 N bis zum Bruch belastet. Die Scherverbundfestigkeit in MPa ergibt sich als Quotient aus der Bruchkraft in N und der Verbundfläche in $mm^2$.

Viskosität:

Die Viskosität wurde mit einem Rheometer des Typs Physica MCR 301 (Anton Paar) im Oszillationsversuch (Platte/Platte) bei 23 °C bestimmt. Der Plattendurchmesser betrug 50 mm und der Spaltabstand 1 mm. Die Methode zur Messung der Viskositäten umfasst vier aufeinanderfolgende Abschnitte.

In Phase I der Messung wird für fünf Minuten (Messpunktdauer 5 s) bei einer Deformation von 0.1% und einer Oszillationsfrequenz von 10 Hz gemessen. Der letzte Punkt der Phase I wird dabei zur Auswertung herangezogen.

In Phase II der Messung wird für 60 s (Messpunktdauer 1 s) bei einer Oszillationsfrequenz von 10 Hz gemessen, wobei die Deformation um 5 %-Punkte pro Sekunde von 0.1% auf 300% erhöht wird.

In Phase III der Messung wird für 60 s (Messpunktdauer 1 s) wieder bei einer Deformation von 0.1% und einer Oszillationsfrequenz von 10 Hz gemessen.

In Phase IV der Messung wird für 4 Minuten (Messpunktdauer 4 s) weiter bei einer Deformation von 0.1% und einer Oszillationsfrequenz von 10 Hz jedoch bei einer größeren Messpunktdauer gemessen. Der letzte Punkt der Phase IV wird dabei zur Auswertung herangezogen.

Dabei beschreibt der Auswertepunkt der Phase I die Viskosität im Ruhezustand und der Auswertepunkt der Phase IV die Viskosität nach erfolgter Scherbelastung (also nach Applikation auf die Zahnoberfläche).

Tabelle 5:

| Beispiel | 3A | 3B | 3C | 3D | 3E | 3F | 3G |
|---|---|---|---|---|---|---|---|
| Haftung (Dentin) [MPa] | 33.2 | 31.8 | 32.2 | 31.7 | 32.5 | 32.3 | 33.3 |
| Haftung (Schmelz) [MPa] | 35.2 | 34.5 | 34.7 | 34.7 | 35.0 | 35.1 | 35.1 |
| Viskosität (I) [Pa*s] | 2.5 | 2.5 | 2.6 | 2.4 | 2.7 | 2.3 | 2.3 |
| Viskosität (IV) [Pa*s] | 2.5 | 2.5 | 2.6 | 2.4 | 2.7 | 2.2 | 2.3 |
| tan δ (I) | 0.89 | 0.83 | 0.85 | 0.82 | 0.90 | 0.90 | 0.83 |
| tan δ (IV) | 0.89 | 0.84 | 0.85 | 0.83 | 0.91 | 0.91 | 0.84 |
| Viskosität (I) (6 Monate, 23 °C) [Pa*s] | 2.5 | 2.5 | 2.5 | 2.4 | 2.7 | 2.2 | 2.2 |
| Viskosität (IV) (6 Monate, 23 °C) [Pa*s] | 2.4 | 2.5 | 2.5 | 2.3 | 2.6 | 2.1 | 2.2 |
| tan δ (I) (6 Monate, 23 °C) | 0.90 | 0.89 | 0.86 | 0.87 | 0.85 | 0.93 | 0.90 |
| tan δ (IV) (6 Monate, 23 °C) | 0.90 | 0.90 | 0.88 | 0.90 | 0.87 | 0.96 | 0.90 |

Tabelle 5 (Fortsetzung):

| Beispiel | 3H | 3I | 3J | 3K | 3L | 3M | 3N |
|---|---|---|---|---|---|---|---|
| Haftung (Dentin) [MPa] | 32.3 | 31.1 | 30.7 | 33.6 | 31.2 | 33.1 | 32.1 |
| Haftung (Schmelz) [MPa] | 35.2 | 35.5 | 34.6 | 34.8 | 35.5 | 34.5 | 34.9 |
| Viskosität (I) [Pa*s] | 2.4 | 2.1 | 2.4 | 2.3 | 2.6 | 2.8 | 2.9 |
| Viskosität (IV) [Pa*s] | 2.4 | 2.0 | 2.3 | 2.3 | 2.5 | 2.7 | 2.8 |
| tan δ (I) | 0.88 | 0.89 | 0.87 | 0.87 | 0.81 | 0.79 | 0.88 |
| tan δ (IV) | 0.90 | 0.90 | 0.89 | 0.88 | 0.83 | 0.81 | 0.90 |
| Viskosität (I) (6 Monate, 23 °C) [Pa*s] | 2.4 | 2.0 | 2.3 | 2.3 | 2.6 | 2.7 | 2.8 |
| Viskosität (IV) (6 Monate, 23 °C) [Pa*s] | 2.3 | 2.0 | 2.3 | 2.2 | 2.5 | 2.6 | 2.7 |
| tan δ (I) (6 Monate, 23 °C) | 0.88 | 0.90 | 0.89 | 0.88 | 0.82 | 0.80 | 0.88 |
| tan δ (IV) (6 Monate, 23 °C) | 0.89 | 0.91 | 0.91 | 0.89 | 0.85 | 0.81 | 0.89 |

Tabelle 5 (Fortsetzung):

| Beispiel | 3O | 3P | 3Q | 3R | 3S | 3T | 3U |
|---|---|---|---|---|---|---|---|
| Haftung (Dentin) [MPa] | 31.3 | 31.6 | 32.7 | 30.1 | 32.4 | 32.1 | 30.0 |
| Haftung (Schmelz) [MPa] | 34.9 | 35.3 | 35.4 | 33.9 | 33.8 | 34.1 | 33.1 |
| Viskosität (I) [Pa*s] | 1.8 | 1.3 | 1.1 | 1.9 | 2.1 | 3.5 | 4.6 |
| Viskosität (IV) [Pa*s] | 1.7 | 1.3 | 1.1 | 1.8 | 2.0 | 3.2 | 4.3 |
| tan δ (I) | 0.91 | 0.97 | 0.99 | 0.90 | 0.87 | 0.75 | 0.91 |
| tan δ (IV) | 0.92 | 0.98 | 0.99 | 0.92 | 0.89 | 0.78 | 0.92 |
| Viskosität (I) (6 Monate, 23 °C) [Pa*s] | 1.7 | 1.4 | 1.2 | 1.8 | 2.0 | 3.4 | 4.4 |
| Viskosität (IV) (6 Monate, 23 °C) [Pa*s] | 1.7 | 1.4 | 1.2 | 1.1 | 1.9 | 3.2 | 4.1 |
| tan δ (I) (6 Monate, 23 °C) | 0.91 | 0.95 | 0.96 | 0.90 | 0.86 | 0.79 | 0.91 |
| tan δ (IV) (6 Monate, 23 °C) | 0.92 | 0.96 | 0.98 | 0.91 | 0.88 | 0.83 | 0.92 |

Tabelle 6:

| Vergleichsbeispiel | 4A | 4B | 4C | 4D | 4E | 4F | 4G |
|---|---|---|---|---|---|---|---|
| Haftung (Dentin) [MPa] | 14.2 | 15.1 | 17.3 | 16.8 | 15.3 | 12.8 | 11.5 |

(fortgesetzt)

| Vergleichsbeispiel | 4A | 4B | 4C | 4D | 4E | 4F | 4G |
|---|---|---|---|---|---|---|---|
| Haftung (Schmelz) [MPa] | 16.3 | 18.4 | 20.4 | 19.1 | 17.7 | 14.3 | 12.3 |
| Viskosität (I) [Pa*s] | 0.2 | 0.3 | 3.0 | 6.0 | 0.6 | 0.8 | 10.0 |
| Viskosität (IV) [Pa*s] | 0.2 | 0.3 | 2.9 | 5.8 | 0.6 | 0.8 | 1.1 |
| tan δ (I) | 1.50 | 1.39 | 0.88 | 0.82 | 1.28 | 1.22 | 1.50 |
| tan δ (IV) | 1.50 | 1.39 | 0.90 | 0.85 | 1.29 | 1.23 | 1.50 |
| Viskosität (I) (6 Monate, 23 °C) [Pa*s] | n.b. | n.b. | 4.2 | 8.3 | n.b. | n.b. | n.b. |
| Viskosität (IV) (6 Monate, 23 °C) [Pa*s] | n.b. | n.b. | 4.1 | 8.0 | n.b. | n.b. | n.b. |
| tan δ (I) (6 Monate, 23 °C) | n.b. | n.b. | 0.81 | 0.75 | n.b. | n.b. | n.b. |
| tan δ (IV) (6 Monate, 23 °C) | n.b. | n.b. | 0.84 | 0.79 | n.b. | n.b. | n.b. |

Tabelle 6 (Fortsetzung):

| Vergleichsbeispiel | 4H | 4I | 4J | 4K | 4L | 4M | 4N |
|---|---|---|---|---|---|---|---|
| Haftung (Dentin) [MPa] | 20.4 | 20.8 | n.b. | n.b. | n.b. | 18.4 | 19.7 |
| Haftung (Schmelz) [MPa] | 22.3 | 22.5 | n.b. | n.b. | n.b. | 19.8 | 20.5 |
| Viskosität (I) [Pa*s] | 1.2 | 6.5 | 0.2 | 0.2 | 0.3 | 2.5 | 8.2 |
| Viskosität (IV) [Pa*s] | 1.1 | 6.2 | 0.2 | 0.2 | 0.3 | 2.3 | 7.9 |
| tan δ (I) | 1.12 | 0.91 | 1.51 | 1.48 | 1.41 | 0.97 | 1.12 |
| tan δ (IV) | 1.14 | 0.94 | 1.51 | 1.48 | 1.41 | 0.99 | 1.14 |
| Viskosität (I) (6 Monate, 23 °C) [Pa*s] | 0.9 | 1.8 | n.b. | n.b. | n.b. | 1.1 | 1.8 |
| Viskosität (IV) (6 Monate, 23 °C) [Pa*s] | 0.9 | 1.4 | n.b. | n.b. | n.b. | 1.0 | 1.6 |
| tan δ (I) (6 Monate, 23 °C) | 1.18 | 1.02 | n.b. | n.b. | n.b. | 1.13 | 1.03 |
| tan δ (IV) (6 Monate, 23 °C) | 1.19 | 1.05 | n.b. | n.b. | n.b. | 1.14 | 1.05 |

Tabelle 6 (Fortsetzung):

| Vergleichsbeispiel | 4O | 4P |
|---|---|---|
| Haftung (Dentin) [MPa] | 28.9 | 25.3 |
| Haftung (Schmelz) [MPa] | 30.7 | 28.4 |
| Viskosität (I) [Pa*s] | 60.4 | 58.8 |
| Viskosität (IV) [Pa*s] | 58.9 | 58.4 |
| tan δ (I) | 0.65 | 0.78 |
| tan δ (IV) | 0.88 | 0.83 |
| Viskosität (I) (6 Monate, 23 °C) [Pa*s] | 22.4 | 15.7 |
| Viskosität (IV) (6 Monate, 23 °C) [Pa*s] | 20.1 | 15.3 |
| tan δ (I) (6 Monate, 23 °C) | 0.54 | 0.69 |
| tan δ (IV) (6 Monate, 23 °C) | 0.68 | 0.75 |

## Patentansprüche

**1.** Dentale Ätzzusammensetzung umfassend

A) Phosphorsäure in einer Menge von 10 bis 45 Gew.-%,
B) Wasser in einer Menge von 30 bis 60 Gew.-%,
C) eine oder mehrere Urethan-Harnstoff-Verbindungen in einer Menge von 5 bis 20 Gew.-%,
D) ein oder mehrere Wasser mischbaren Lösungsmittel in einer Menge von 0 bis 20 Gew.-%, und
E) Farbmittel in einer Menge von 0 bis 5 Gew.-%,
jeweils bezogen auf die Gesamtzusammensetzung,
**dadurch gekennzeichnet, dass** die Urethan-Harnstoff-Verbindungen (C) der Formel

$$R^1\text{-O-C(=O)-NH-}R^2\text{-NH-C(=O)[-NH-}R^3\text{-NH-C(=O)-NH-}R^2\text{-NH-C(=O)]}_x\text{-O}R^1$$

entsprechen, worin
$R^1$ für einen n-Alkyl-Rest mit 4 bis 22 C-Atomen, einen verzweigten Alkyl-Rest mit 4 bis 22 C-Atomen, einen Alkenylrest mit 3 bis 18 C-Atomen, einen Cycloalkylrest mit 3 bis 20 C-Atomen, einen Arylrest mit 6 bis 12 C-Atomen, einen Arylalkylrest mit 7 bis 12 C-Atomen, einen Rest der Formel $C_mH_{2m+1}(O\text{-}C_nH_{2n})_p$-, $C_mH_{2m+1}(OOC\text{-}C_vH_{2v})_p$- oder $R^5\text{-}C_6H_4(O\text{-}C_nH_{2n})_p$- mit m = 1 bis 22, n = 2 bis 4, p = 1 bis 15, v = 4 oder 5 und $R^5$ für einen Alkylrest mit 1 bis 12 C-Atomen steht, wobei unterschiedliche Reste $R^1$ gleich oder verschieden sein können,
$R^2$ für einen verzweigten oder unverzweigten Alkylen-Rest mit 4 bis 22 C-Atomen, Alkenylen-Rest mit 3 bis 18 C-Atomen, Alkinylen-Rest mit 2 bis 20 C-Atomen, Cycloalkylen-Rest mit 3 bis 20 C-Atomen, Cycloalkenylen-Rest mit 3 bis 20 C-Atomen, Arylen-Rest mit 6 bis 12 C-Atomen oder Arylalkylen-Rest mit 7 bis 14 C-Atomen steht, wobei unterschiedliche Reste $R^2$ gleich oder verschieden sein können,
$R^3$ für

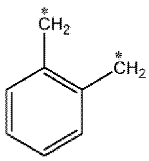 , 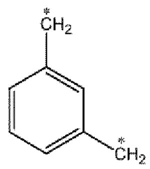 , 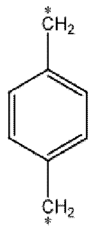 , 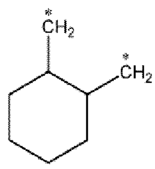 , 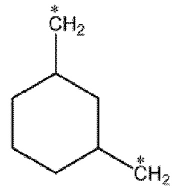 , 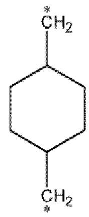 ,

oder

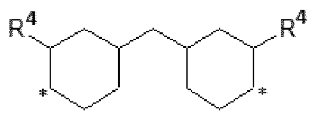

mit $R^4$ = $CH_3$ oder H, wobei unterschiedliche Reste $R^3$ gleich oder verschieden sein können, und
x für eine ganze Zahl von 1 bis 100 steht.

**2.** Dentale Ätzzusammensetzung nach Anspruch 1, wobei die Urethan-Harnstoff-Verbindungen (C) der Formel

$$R^1\text{-O-C(=O)-NH-}R^2\text{-NH-C(=O)[-NH-}R^3\text{-NH-C(=O)-NH-}R^2\text{-NH-C(=O)]}_x\text{-O}R^1$$

entsprechen, worin
$R^1$ für einen n-Alkyl-Rest mit 4 bis 10 C-Atomen, einen verzweigten Alkyl-Rest mit 4 bis 10 C-Atomen, einen Alkenylrest mit 3 bis 10 C-Atomen, einen Cycloalkylrest mit 3 bis 10 C-Atomen, einen Arylrest mit 6 bis 12 C-Atomen, einen Arylalkylrest mit 7 bis 12 C-Atomen, einen Rest der Formel $C_mH_{2m+1}(O\text{-}C_nH_{2n})_p$-, $C_mH_{2m+1}(OOC\text{-}C_vH_{2v})_p$- oder $R^5\text{-}C_6H_4(O\text{-}C_nH_{2n})_p$- mit m = 1 bis 10, n = 2 bis 4, p = 1 bis 15, v = 4 oder 5 und $R^5$ für einen Alkylrest mit 1 bis 12 C-Atomen, bevorzugt für einen n-Alkyl-Rest mit 4 bis 10 C-Atomen, einen verzweigten Alkyl-Rest mit 4 bis 10 C-Atomen, einen Rest der Formel $C_mH_{2m+1}(O\text{-}C_nH_{2n})_p$- oder $C_mH_{2m+1}(OOC\text{-}C_vH_{2v})_p$- mit m = 1 bis 10, n = 2 bis 4, p = 1 bis 15 und v = 4 oder 5, steht, wobei unterschiedliche Reste $R^1$ gleich oder verschieden sein können,
$R^2$ für

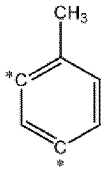 , 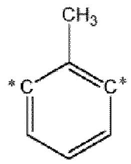 , 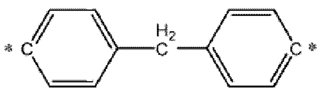 , 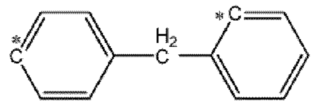 ,

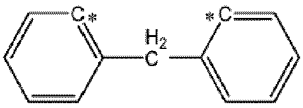 , 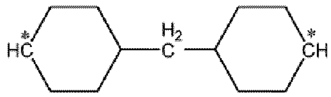 , 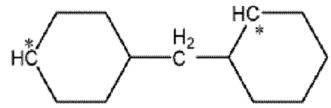 ,

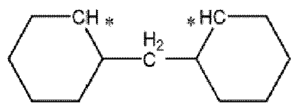

oder $-(CH_2)_w-$ mit w = 2 bis 10, bevorzugt für

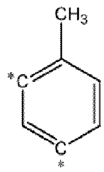

oder

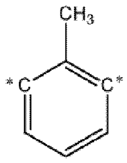 ,

steht, wobei unterschiedliche Reste $R^2$ gleich oder verschieden sein können,
$R^3$ für

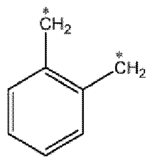 , 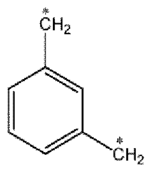 oder 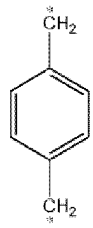

steht, wobei unterschiedliche Reste $R^3$ gleich oder verschieden sein können, und
x für eine ganze Zahl von 1 bis 20, bevorzugt 1 bis 10, steht.

**3.** Dentale Ätzzusammensetzung nach einem der vorangehenden Ansprüche, wobei die mit Wasser mischbaren Lösungsmittel (D) ausgewählt sind aus der Gruppe bestehend aus Ethanol, Propan-1-ol, Propan-2-ol, Butan-1-ol, Butan-2-ol, 2-Methylpropan-1-ol, 2-Methylpropan-2-ol, Glycerin, Ethylenglycol, Propylenglycol, Butylenglycol, Diethylenglycol, Polyethylenglycol, Polypropylenglycol, 2-Butoxyethan-1-ol, DMSO und Aceton, vorzugsweise ausgewählt sind aus der Gruppe bestehend aus Ethanol, Propan-1-ol, Propan-2-ol, Glycerin, Polyethylenglycol, Polypropylenglycol und DMSO.

**4.** Dentale Ätzzusammensetzung nach einem der vorangehenden Ansprüche, wobei die Farbmittel (E) ausgewählt sind aus der Gruppe bestehend aus Farbstoffen, organischen Farbpigmenten und anorganischen Farbpigmenten,

bevorzugt aus Farbstoffen, besonders bevorzugt aus Phenothiazin-Farbstoffen,
und/oder
wobei es sich bei den Farbmitteln (E) um blaue, grüne oder rote, bevorzugt um blaue, Farbmittel handelt.

**5.** Dentale Ätzzusammensetzung nach einem der vorangehenden Ansprüche umfassend

A) in einer Menge von 30 bis 42 Gew.-%,
B) in einer Menge von 40 bis 60 Gew.-%,
C) in einer Menge von 5 bis 15 Gew.-%,
D) in einer Menge von 1 bis 15 Gew.-%, und
E) in einer Menge von 0,0001 bis 1 Gew.-%,

jeweils bezogen auf die Gesamtzusammensetzung.

**6.** Dentale Ätzzusammensetzung nach einem der vorangehenden Ansprüche die im Wesentlichen frei von pyrogener Kieselsäure, bevorzugt im Wesentlichen frei von Silicapartikeln, besonders bevorzugt im Wesentlichen frei von anorganischen Feststoffen ist
und/oder
die außer A), B), C), D) und E) keine weiteren Bestandteile enthält.

**7.** Dentale Ätzzusammensetzung nach einem der vorangehenden Ansprüche, die einen Verlustfaktor tan $\delta$ von kleiner 1 und/oder eine Viskosität im Bereich von 0.1 bis 200 Pa*s, bevorzugt von 0.5 bis 150 Pa*s, besonders bevorzugt von 1 bis 100 Pa*s, ganz besonders bevorzugt von 1 bis 50 Pa*s aufweist und die vorzugsweise auch nach Lagerung über 6 Monate bei 23 °C einen Verlustfaktor tan $\delta$ von kleiner 1 und/oder eine Viskosität im Bereich von 0.1 bis 200 Pa*s, bevorzugt von 0.5 bis 150 Pa*s, besonders bevorzugt von 1 bis 100 Pa*s, ganz besonders bevorzugt von 1 bis 50 Pa*s aufweist, wobei Verlustfaktor tan $\delta$ und Viskosität wie in der Beschreibung beschrieben gemessen werden.

**8.** Dentale Ätzzusammensetzung nach einem der Ansprüche 1 bis 7 zur Anwendung in einem therapeutischen Verfahren zum Ätzen der Zahnhartsubstanz im Rahmen der Füllungstherapie,
vorzugsweise in einem therapeutischen Verfahren umfassend die Schritte

i) optional Trockenlegung der zu behandelnden Zähne, vorzugsweise mit Kofferdam,
ii) Auftragen einer dentalen Ätzzusammensetzung nach einem der Ansprüche 1 bis 7 auf die zu behandelnde Zahnhartsubstanz,
iii) Einwirken lassen der dentalen Ätzzusammensetzung unter Erzielung einer Ätzwirkung an der Zahnhartsubstanz,
iv) Abspülen der dentalen Ätzzusammensetzung,
v) Auftragen einer dentalen Primer- und/oder Adhäsivzusammensetzung auf die geätzte Zahnhartsubstanz,
vi) optional Polymerisation der dentalen Primer- und/oder Adhäsivzusammensetzung,
vii) Auftragen einer dentalen Restaurationszusammensetzung und
viii) Polymerisation der dentalen Restaurationszusammensetzung.

**9.** Kit enthaltend

- eine dentale Ätzzusammensetzung nach einem der Ansprüche 1 bis 7,
- eine dentale Primer- und/oder Adhäsivzusammensetzung und
- optional eine dentale Restaurationszusammensetzung.

## Claims

**1.** Dental etching composition comprising

A) phosphoric acid, in an amount of 10% to 45% by weight,
B) water, in an amount of 30% to 60% by weight,
C) one or more urethane-urea compounds, in an amount of 5% to 20% by weight,
D) one or more solvents miscible with water, in an amount of 0% to 20% by weight,
E) colourant, in an amount of 0% to 5% by weight, based in each case on the overall composition,

**characterized in that** the urethane-urea compounds (C) conform to the formula

$$R^1\text{-O-C(=O)-NH-}R^2\text{-NH-C(=O)[-NH-}R^3\text{-NH-C(=O)-NH-}R^2\text{-NH-C(=O)]}_x\text{-O}R^1$$

in which

$R^1$ is an n-alkyl radical having 4 to 22 carbon atoms, a branched alkyl radical having 4 to 22 carbon atoms, an alkenyl radical having 3 to 18 carbon atoms, a cycloalkyl radical having 3 to 20 carbon atoms, an aryl radical having 6 to 12 carbon atoms, an arylalkyl radical having 7 to 12 carbon atoms, a radical of the formula $C_mH_{2m+1}(O\text{-}C_nH_{2n})p$-, $C_mH_{2m+1}(OOC\text{-}C_vH_{2v})_p$- or $R^5\text{-}C_6H_4(O\text{-}C_nH_{2n})_p$- with m = 1 to 22, n = 2 to 4, p = 1 to 15, v = 4 or 5, and $R^5$ is an alkyl radical having 1 to 12 carbon atoms, where different $R^1$ radicals may be the same or different,

$R^2$ is a branched or unbranched alkylene radical having 4 to 22 carbon atoms, alkenylene radical having 3 to 18 carbon atoms, alkynylene radical having 2 to 20 carbon atoms, cycloalkylene radical having 3 to 20 carbon atoms, cycloalkenylene radical having 3 to 20 carbon atoms, arylene radical having 6 to 12 carbon atoms or arylalkylene radical having 7 to 14 carbon atoms, where different $R^2$ radicals may be the same or different,

$R^3$ is

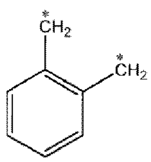,
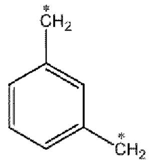,
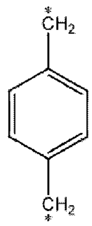,
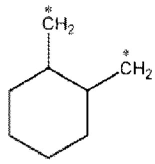,
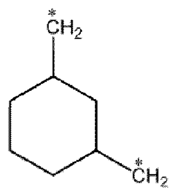,
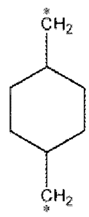

or

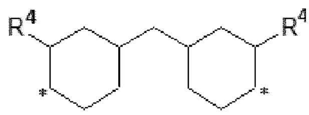

with $R^4 = CH_3$ or H, where different $R^3$ radicals may be the same or different, and
x is an integer from 1 to 100.

**2.** Dental etching composition according to Claim 1,

wherein the urethane-urea compounds (C) conform to the formula

$$R^1\text{-O-C(=O)-NH-}R^2\text{-NH-C(=O)[-NH-}R^3\text{-NH-C(=O)-NH-}R^2\text{-NH-C(=O)]}_x\text{-O}R^1$$

in which

$R^1$ is an n-alkyl radical having 4 to 10 carbon atoms, a branched alkyl radical having 4 to 10 carbon atoms, an alkenyl radical having 3 to 10 carbon atoms, a cycloalkyl radical having 3 to 10 carbon atoms, an aryl radical having 6 to 12 carbon atoms, an arylalkyl radical having 7 to 12 carbon atoms, a radical of the formula $C_mH_{2m+1}(O\text{-}C_nH_{2n})_p$-, $C_mH_{2m+1}(OOC\text{-}C_vH_{2v})_p$- or $R^5\text{-}C_6H_4(O\text{-}C_nH_{2n})p$- with m = 1 to 10, n = 2 to 4, p = 1 to 15, v = 4 or 5, and $R^5$ is an alkyl radical having 1 to 12 carbon atoms, preferably an n-alkyl radical having 4 to 10 carbon atoms, a branched alkyl radical having 4 to 10 carbon atoms, a radical of the formula $C_mH_{2m+1}(O\text{-}C_nH_{2n})_p$- or $C_mH_{2m+1}(OOC\text{-}C_vH_{2v})_p$- with m = 1 to 10, n = 2 to 4, p = 1 to 15 and v = 4 or 5, where different $R^1$ radicals may be the same or different,

$R^2$ is

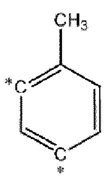,
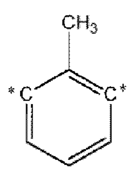,
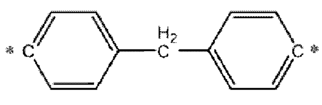,
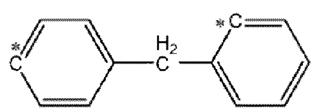

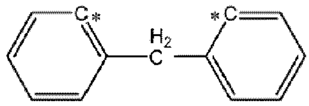 , 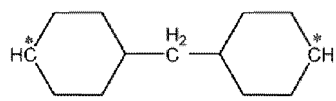 ,

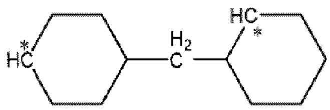 , 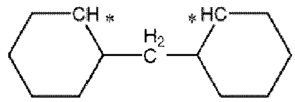

or $-(CH_2)_w-$ with w = 2 to 10, preferably

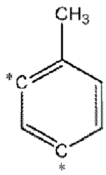 or 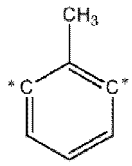 ,

where different $R^2$ radicals may be the same or different,
$R^3$ is

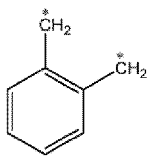 , 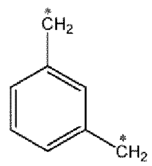 or 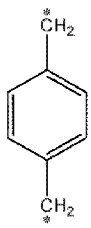 ,

where different $R^3$ radicals may be the same or different, and
x is an integer from 1 to 20, preferably 1 to 10.

3. Dental etching composition according to any of the preceding claims, wherein the water-miscible solvents (D) are selected from the group consisting of ethanol, propan-1-ol, propan-2-ol, butan-1-ol, butan-2-ol, 2-methylpropan-1-ol, 2-methylpropan-2-ol, glycerol, ethylene glycol, propylene glycol, butylene glycol, diethylene glycol, polyethylene glycol, polypropylene glycol, 2-butoxyethan-1-ol, DMSO and acetone, preferably selected from the group consisting of ethanol, propan-1-ol, propan-2-ol, glycerol, polyethylene glycol, polypropylene glycol and DMSO.

4. Dental etching composition according to any of the preceding claims, wherein the colourants (E) are selected from the group consisting of dyes, organic colour pigments and inorganic colour pigments, preferably from dyes, more preferably from phenothiazine dyes,
and/or
wherein the colourants (E) are blue, green or red, preferably blue, colourants.

5. Dental etching composition according to any of the preceding claims, comprising

A) in an amount of 30% to 42% by weight,
B) in an amount of 40% to 60% by weight,
C) in an amount of 5% to 15% by weight,
D) in an amount of 1% to 15% by weight and
E) in an amount of 0.0001% to 1% by weight,

based in each case on the overall composition.

6. Dental etching composition according to any of the preceding claims which is essentially free of fumed silica, preferably essentially free of silica particles, more preferably essentially free of inorganic solids,

and/or
which does not contain any further constituents apart from A), B), C), D) and E).

**7.** Dental etching composition according to any of the preceding claims which has a loss factor tan $\delta$ of less than 1 and/or a viscosity in the range from 0.1 to 200 Pa*s, preferably from 0.5 to 150 Pa*s, more preferably from 1 to 100 Pa*s, most preferably from 1 to 50 Pa*s, and which preferably even after storage at 23°C for 6 months has a loss factor tan $\delta$ of less than 1 and/or a viscosity in the range from 0.1 to 200 Pa*s, preferably from 0.5 to 150 Pa*s, more preferably from 1 to 100 Pa*s, most preferably from 1 to 50 Pa*s, wherein loss factor tan $\delta$ and viscosity are measured as described in the description.

**8.** Dental etching composition according to any of Claims 1 to 7 for use in a therapeutic method for etching the hard substance of the tooth in the course of filling treatment,
preferably in a therapeutic method comprising the steps of

i) optionally desiccating the teeth to be treated, preferably with a dental dam,
ii) applying a dental etching composition according to any of Claims 1 to 7 to the hard substance of the tooth to be treated,
iii) allowing a contact time of the dental etching composition to achieve an etching effect on the hard substance of the tooth,
iv) rinsing off the dental etching composition,
v) applying a dental primer composition and/or adhesive composition to the etched hard substance of the tooth,
vi) optionally polymerizing the dental primer composition and/or adhesive composition,
vii) applying a dental restoration composition and
viii) polymerizing the dental restoration composition.

**9.** Kit comprising

- a dental etching composition according to any of Claims 1 to 7,
- a dental primer composition and/or adhesive composition and
- optionally a dental restoration composition.

## Revendications

**1.** Composition de gravure dentaire comprenant

A) de l'acide phosphorique en une quantité de 10 à 45% en poids,
B) de l'eau en une quantité de 30 à 60% en poids,
C) un ou plusieurs composés d'uréthane-urée en une quantité de 5 à 20% en poids,
D) un ou plusieurs solvants miscibles à l'eau en une quantité de 0 à 20% en poids,
(E) des colorants en une quantité de 0 à 5% en poids,
à chaque fois par rapport à la composition totale, **caractérisée en ce que** les composés d'uréthane-urée (C) correspondent à la formule

$$R^1\text{-O-C(=O)-NH-}R^2\text{-NH-C(=O)[-NH-}R^3\text{-NH-C(=O)-NH-}R^2\text{-NH-C(=O)]}_x\text{-O}R^1$$

dans laquelle
$R^1$ représente un radical n-alkyle comprenant 4 à 22 atomes de carbone, un radical alkyle ramifié comprenant 4 à 22 atomes de carbone, un radical alcényle comprenant 3 à 18 atomes, un radical cycloalkyle comprenant 3 à 20 atomes de carbone, un radical aryle comprenant 6 à 12 atomes de carbone, un radical arylalkyle comprenant 7 à 12 atomes de carbone, un radical de formule $C_mH_{2m+1}(O\text{-}C_nH_{2n})_p$-, $C_mH_{2m+1}$ $(OOC\text{-}C_vH_{2v})_p$- ou $R^5\text{-}C_6H_4(O\text{-}C_nH_{2n})_p$- où m = 1 à 22, n = 2 à 4, p = 1 à 15, v = 4 ou 5 et $R^5$ représente un radical alkyle comprenant 1 à 12 atomes de carbone, différents radicaux $R^1$ pouvant être identiques ou différents,
$R^2$ représente un radical alkylène ramifié ou non ramifié, comprenant 4 à 22 atomes de carbone, un radical alcénylène comprenant 3 à 18 atomes de carbone, un radical alcynylène comprenant 2 à 20 atomes de carbone, un radical cycloalkylène comprenant 3 à 20 atomes de carbone, un radical cycloalcénylène comprenant 3 à 20 atomes de carbone, un radical arylène comprenant 6 à 12 atomes de carbone ou un radical arylalkylène comprenant 7 à 14 atomes de carbone, différents radicaux $R^2$ pouvant être identiques ou différents,
$R^3$ représente

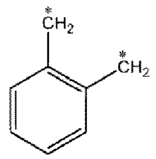 , 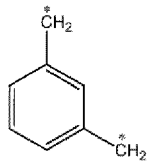 , 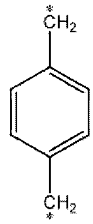 , 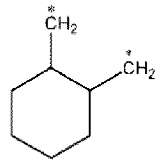 ,

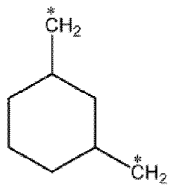 , 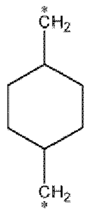 ou 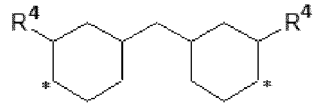 ,

où $R^4 = CH_3$ ou H, différents radicaux $R^3$ pouvant être identiques ou différents et x représente un nombre entier de 1 à 100.

**2.** Composition de gravure dentaire selon la revendication 1, le composé d'uréthane-urée (C) correspondant à la formule

$$R^1\text{-O-C(=O)-NH-}R^2\text{-NH-C(=O)[-NH-}R^3\text{-NH-C(=O)-NH-}R^2\text{-NH-C(=O)]}_x\text{-O}R^1$$

dans laquelle

$R^1$ représente un radical n-alkyle comprenant 4 à 10 atomes de carbone, un radical alkyle ramifié comprenant 4 à 10 atomes de carbone, un radical alcényle comprenant 3 à 10 atomes de carbone, un radical cycloalkyle comprenant 3 à 10 atomes de carbone, un radical aryle comprenant 6 à 12 atomes de carbone, un radical arylalkyle comprenant 7 à 12 atomes de carbone, un radical de formule $C_mH_{2m+1}(OC_nH_{2n})p$-, $C_mH_{2m+1}(OOC\text{-}C_vH_{2v})_p$- ou $R^5\text{-}C_6H_4(O\text{-}C_nH_{2n})_p$- où m = 1 à 10, n = 2 à 4, p = 1 à 15, v = 4 ou 5 et $R^5$ représente un radical alkyle comprenant 1 à 12 atomes de carbone, de préférence un radical n-alkyle comprenant 4 à 10 atomes de carbone, un radical alkyle ramifié comprenant 4 à 10 atomes de carbone, un radical de formule $C_mH_{2m+1}(O\text{-}C_nH_{2n})_p$- ou $C_mH_{2m+1}(OOC\text{-}C_vH_{2v})_p$- où m = 1 à 10, n = 2 à 4, p = 1 à 15 et v = 4 ou 5, différents radicaux $R^1$ pouvant être identiques ou différents,

$R^2$ représente

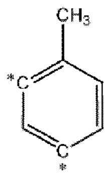 , 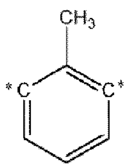 , 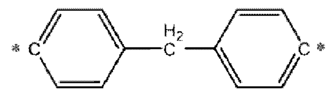 ,

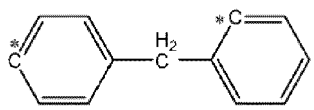 , 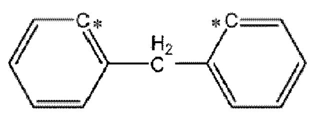 , 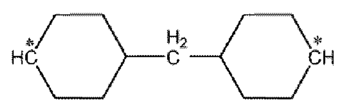

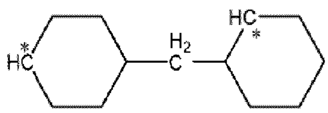 , 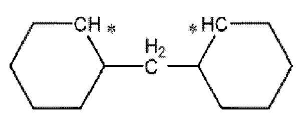

ou $-(CH_2)_w$- où w = 2 à 10, de préférence

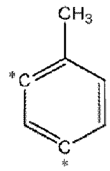 ou 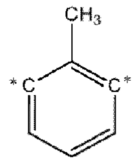 ,

différents radicaux $R^2$ pouvant être identiques ou différents,
$R^3$ représente

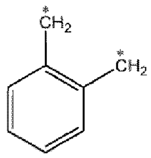 , 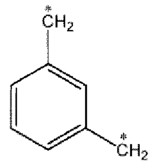 ou 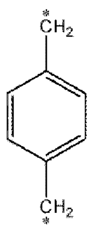 ,

différents radicaux $R^3$ pouvant être identiques ou différents, et
x représente un nombre entier de 1 à 20, de préférence de 1 à 10.

**3.** Composition de gravure dentaire selon l'une des revendications précédentes, les solvants miscibles à l'eau (D) étant choisis dans le groupe constitué par éthanol, propan-1-ol, propan-2-ol, butan-1-ol, butan-2-ol, 2-méthylpropan-1-ol, 2-méthylpropan-2-ol, glycérol, éthylèneglycol, propylèneglycol, butylèneglycol, diéthylèneglycol, polyéthylèneglycol, polypropylèneglycol, 2-butoxyéthan-1-ol, DMSO et acétone, de préférence choisis dans le groupe constitué par éthanol, propan-1-ol, propan-2-ol, glycérol, polyéthylèneglycol, polypropylèneglycol et DMSO.

**4.** Composition de gravure dentaire selon l'une des revendications précédentes, les colorants (E) étant choisis dans le groupe constitué par les colorants, les pigments colorés organiques et les pigments colorés inorganiques, de préférence parmi les colorants, de manière particulièrement préférée parmi les colorants de phénothiazine,
et/ou
les colorants (E) étant des colorants bleus, verts ou rouges, de préférence bleus.

**5.** Composition de gravure dentaire selon l'une des revendications précédentes, comprenant

A) en une quantité de 30 à 42% en poids,
B) en une quantité de 40 à 60% en poids,
C) en une quantité de 5 à 15% en poids,
D) une quantité de 1 à 15% en poids et
E) en une quantité de 0,0001 à 1% en poids,

à chaque fois par rapport à la composition totale.

**6.** Composition de gravure dentaire selon l'une des revendications précédentes, qui est sensiblement exempte de silice pyrogène, de préférence sensiblement exempte de particules de silice, de manière particulièrement préférée sensiblement exempte de solides inorganiques
et/ou
qui ne contient pas d'autres constituants en dehors de A), B), C), D) et E).

**7.** Composition de gravure dentaire selon l'une des revendications précédentes, qui présente un facteur de perte tan $\delta$ inférieur à 1 et/ou une viscosité dans la plage de 0,1 à 200 Pa*s, de préférence de 0,5 à 150 Pa*s, de manière particulièrement préférée de 1 à 100 Pa*s, de manière tout particulièrement préférée de 1 à 50 Pa*s et qui présente de préférence, également après un entreposage pendant 6 mois à 23°C, un facteur de perte tan $\delta$ inférieur à 1 et/ou une viscosité dans la plage de 0,1 à 200 Pa*s, de préférence de 0,5 à 150 Pa*s, de manière particulièrement préférée de 1 à 100 Pa*s, de manière tout particulièrement préférée de 1 à 50 Pa*s, le facteur de perte tan $\delta$ et la viscosité étant mesurés comme décrit dans la description.

**8.** Composition de gravure dentaire selon l'une des revendications 1 à 7 pour une utilisation dans une méthode thérapeutique pour la gravure de la substance dentaire dure dans le cadre de la thérapie de restauration,
de préférence dans une méthode thérapeutique comprenant les étapes

i) séchage éventuel des dents à traiter, de préférence à l'aide d'une digue dentaire,
ii) application d'une composition de gravure dentaire selon l'une des revendications 1 à 7 sur la substance dentaire dure à traiter,
iii) action de la composition de gravure dentaire avec obtention d'un effet de gravure sur la substance dentaire dure,
iv) élimination par rinçage de la composition de gravure dentaire,
v) application d'une composition d'apprêt et/ou adhésive dentaire sur la substance dentaire dure gravée,
vi) polymérisation éventuelle de la composition d'apprêt et/ou adhésive dentaire,
vii) application d'une composition de restauration dentaire et
viii) polymérisation de la composition de restauration dentaire.

**9.** Kit contenant

- une composition de gravure dentaire selon l'une des revendications 1 à 7,
- une composition d'apprêt et/ou adhésive dentaire et
- éventuellement une composition de restauration dentaire.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 4802950 A
- US 6753001 B2
- US 6537563 B2
- US 5954996 A
- US 20120161067 A1
- US 6312667 B1
- US 6027341 A
- WO 2007131725 A1
- EP 2108356 A1
- US 5722833 A
- WO 2015142392 A1
- EP 0006252 B1
- EP 1048681 B1
- EP 1188779 B1
- EP 1396510 B1
- EP 2370489 B1
- EP 2475699 B1
- EP 3328909 B1